# EUROPÄISCHE PATENTSCHRIFT

(11) **EP 1 015 433 B1**
(45) Veröffentlichungstag und Bekanntmachung des Hinweises auf die Patenterteilung: **26.05.2004**
(21) Anmeldenummer: 98950009.5
(22) Anmeldetag: 09.09.1998
(51) Int. Cl.: C07D 221/26, A61K 31/435, A61K 31/485

(54) **SUBSTITUIERTE 1,2,3,4,5,6-HEXAHYDRO-2,6-METHANO-3-BENZAZOCIN-10-OLE, VERFAHREN ZU IHRER HERSTELLUNG UND IHRE VERWENDUNG ALS ARZNEIMITTEL**
SUBSTITUTED 1,2,3,4,5,6-HEXAHYDRO-2,6-METHANO-3-BENZAZOCINE-10-OLES, METHOD FOR THE PRODUCTION AND USE THEREOF AS MEDICAMENTS
1,2,3,4,5,6-HEXAHYDRO-2,6-METHANO-3-BENZAZOCINE-1O-OLES SUBSTITUES, PROCEDES PERMETTANT DE LES PREPARER ET LEUR UTILISATION COMME MEDICAMENTS

(30) Priorität: 12.09.1997 DE 19740110
(43) Veröffentlichungstag der Anmeldung: 05.07.2000
(73) Patentinhaber: Boehringer Ingelheim Pharma GmbH & Co.KG, 55218 Ingelheim am Rhein (DE)
(72) Erfinder: GRAUERT, Matthias, D-55218 Ingelheim am Rhein (DE); CARTER, Adrian, D-55411 Bingen am Rhein (DE); BECHTEL, Wolf-Dietrich, D-55437 Appenheim (DE); WEISER, Thomas, D-55268 Nieder-Olm (DE); PALLUK, Rainer, D-55411 Bingen am Rhein (DE); PSCHORN, Uwe, D-55126 Mainz (DE)
(74) Vertreter: Laudien, Dieter, Dr.
(86) Internationale Anmeldenummer: PCT/EP1998/005734
(87) Internationale Veröffentlichungsnummer: WO 1999/014199

(56) Entgegenhaltungen:
- EP-A- 0 006 449
- EP-A- 0 521 422
- DE-A- 2 948 308
- DE-A- 19 528 472
- M. GRAUERT ET AL.: JOURNAL OF MEDICINAL CHEMISTRY, Bd. 40, Nr. 18, 1997, Seiten 2922-30, XP002090488
- N. YOKOYAMA ET AL.: JOURNAL OF MEDICINAL CHEMISTRY, Bd. 22, Nr. 5, 1979, Seiten 537-53, XP002090489
- C. P. TAYLOR ET AL.: TRENDS IN PHARMACOLOGICAL SCIENCES, Bd. 16, September 1995, Seiten 309-16, XP002090490 in der Anmeldung erwähnt

## Beschreibung

Die vorliegende Anmeldung betrifft neue, substituierte 1,2,3,4,5,6-Hexahydro-2,6-methano-3-benzazocin-10-ole der allgemeinen Formel I: 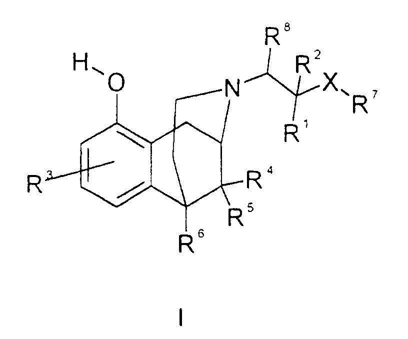 worin
- X: eine Einfachbindung, -O-, C₁-C₄-Alkylen, eine Alkylenbrücke mit 1 bis 8 Kohlenstoffatomen die verzeigt oder unverzweigt sein kann und an beliebiger Stelle in der Brücke ein oder zwei Sauerstoffatom(e) aufweisen kann, vorzugsweise O-C₁-C₃-Alkylen oder -O-CH₂-CH₂-O-, -O-CH₂-CH₂-NH-;
- R¹: Wasserstoff, Methyl, Ethyl, Phenyl;
- R²: Wasserstoff, Methyl;
- R³: Wasserstoff, Fluor, Chlor, Brom, Hydroxy, Methyl, Methoxy;
- R⁴: Wasserstoff, Methyl, Ethyl;
- R⁵: Wasserstoff, Methyl, Ethyl;
- R⁶: Wasserstoff, Methyl, Ethyl;
- R⁷: tert.-Butyl, Cyclohexyl, Phenyl gegebenenfalls substittuiert durch R⁹und R¹⁰, die gleich oder verschieden sein können, 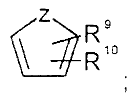
- R⁸: Wasserstoff, C₁-C₄-Alkyl;
- Z: Sauerstoff, NH, Schwefel;
- R⁹: Wasserstoff, Methyl, Fluor, Chlor, Brom, Methoxy;
- R¹⁰: Wasserstoff, Methyl, Fluor, Chlor, Brom, Methoxy;
bedeuten können -
gegebenenfalls in Form der einzelnen optischen Isomeren, Mischungen der einzelnen Enantiomeren oder Racemate sowie in Form der freien Basen oder der enstsprechenden Säureadditionssalze mit pharmakologisch unbedenklichen Säuren wie - wie z.B. Säureadditionssalze mit Halogenwasserstoffsäuren - beispielsweise Chlor- oder Bromwasserstoffsäure -oder entsprechende organische Säuren - beispielsweise Fumar- oder Diglycolsäure.

Bevorzugt sind Verbindungen der allgemeinen Formel I, in welcher in der obigen Definition R⁴ und R⁵ entweder beide gleichzeitg Methyl oder unabhängig voneinander Wasserstoff oder Methyl bedeuten können, wobei wenigstens einer der Substituenten eine Methylgruppe bedeutet;

Bevorzugt sind Verbindungen der allgemeinen Formel I, in der
- X: Sauerstoff;
- R¹: Wasserstoff, Methyl oder Ethyl;
- R²: Wasserstoff,
- R³: Wasserstoff;
- R⁴: Wasserstoff oder Methyl;
- R⁵: Wasserstoff oder Methyl;
- R⁶: Methyl;
- R⁷: Phenyl;
- R⁸: Wasserstoff
- R⁹ und R¹⁰: unabhängig voneinander Wasserstoff, Methyl, Fluor oder Methoxy
bedeuten können-
insbesondere Verbindungen der allgemeinen Formel I, in welcher in der obigen Definition R⁴ und R⁵ entweder beide gleichzeitg Methyl oder unabhängig voneinander Wasserstoff oder Methyl bedeuten können, wobei wenigstens einer der Substituenten eine Methylgruppe bedeutet;

Ganz besonders bevorzugt sind folgende Verbindungen:
(-)-(1R,2"S)-2-(2"-Benzyloxy)propyl-4'-hydroxy-5,9,9-trimethyl-6,7-benzomorphan und
(-)-(1R,2"S)-2-[2"-(2'",6'"-Difluorobenzyl)oxy]propyl-4'-hydroxy-5,9,9-trimethyl-6,7-benzomorphan
in Form der freien Basen oder der enstsprechenden Säureadditionssalze mit pharmakologisch unbedenklichen Säuren.

Soweit nicht im einzelnen abweichende Angaben gemacht werden, werden die allgemeinen Definitionen im folgenden Sinn gebraucht:
C₁-C₄-Alkyl bzw. C₁-C₈-Alkyl steht im allgemeinen für einen verzweigten oder unverzweigten Kohlenwasserstoffrest mit 1 bis 4 bzw. 1 bis 8 Kohlenstoffatom(en), der gegebenenfalls mit einem oder mehreren Halogenatom(en) - vorzugsweise Fluor - substituiert sein kann, die untereinander gleich oder verschieden sein können. Als Beispiele seien folgende Kohlenwasserstoffreste genannt:
   Methyl, Ethyl, Propyl, 1-Methylethyl (Isopropyl), n-Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2,-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl. Bevorzugt sind - sofern nicht anders angegeben - Niederalkylreste mit 1 bis 4 Kohlenstoffatomen, wie Methyl, Ethyl, Propyl, iso-Propyl, n-Butyl, 1-Methylpropyl, 2-Methylpropyl oder 1,1-Dimethylethyl.

Entsprechend bedeutet Alkylen eine verzweigte oder unverzweigte zweibindige Kohlenwasserstoffbrücke mit 1 bis 8 Kohlenstoffatomen, die gegebenenfalls mit einem oder mehreren Halogenatom(en) - vorzugsweise Fluor - substituiert sein kann, die untereinander gleich oder verschieden sein können.

Alkoxy steht im allgemeinen für einen über ein Sauerstoffatom gebundenen geradkettigen oder verzweigten Kohlenwasserstoffrest - bevorzugt ist ein Niederalkoxyrest mit 1 bis 4 Kohlenstoffatom(en). - Besonders bevorzugt ist die Methoxygruppe.

### Herstellungsverfahren

Die erfindungsgemäßen Verbindungen lassen sich nach aus dem Stand der Technik bekannten Verfahren herstellen [WO 97/06146]. Gegenstand der Erfindung sind die enantiomerenreinen Verbindungen sowie die jeweiligen Racemate.

Schlüsselverbindungen sind die Nor-Benzomorphane **2a** bis **5a**, die im Schema als die entsprechenden (-)-Enantiomere dargestellt werden:

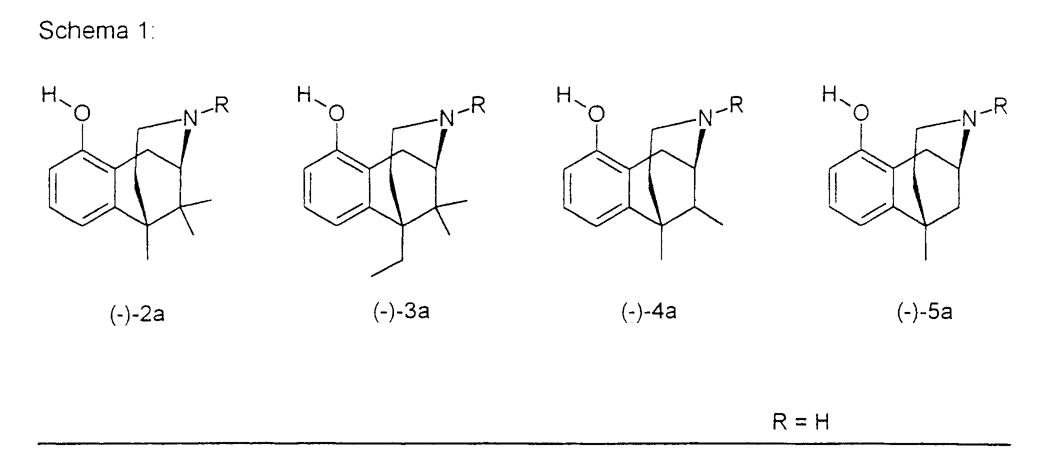

Die Synthese von (-)-2a ist für R=H in der Deutschen Offenlegungsschrift Nr.: 195 28 472 beschrieben.

Die Verbindung **3** kann in Analogie zu Verbindung **2** hergestellt werden. Ausgangsverbindung ist das bei der Synthese von **2** als Zwischenprodukt anfallende Piperidon **6**, das beispielsweise mit einem Ethyltriphenylphosphonium-Salz statt mit dem entsprechenden Methyl-Derivat - wie aus dem Stand der Technik bereits bekannt - umgestezt wird (vgl. Schema 2!).

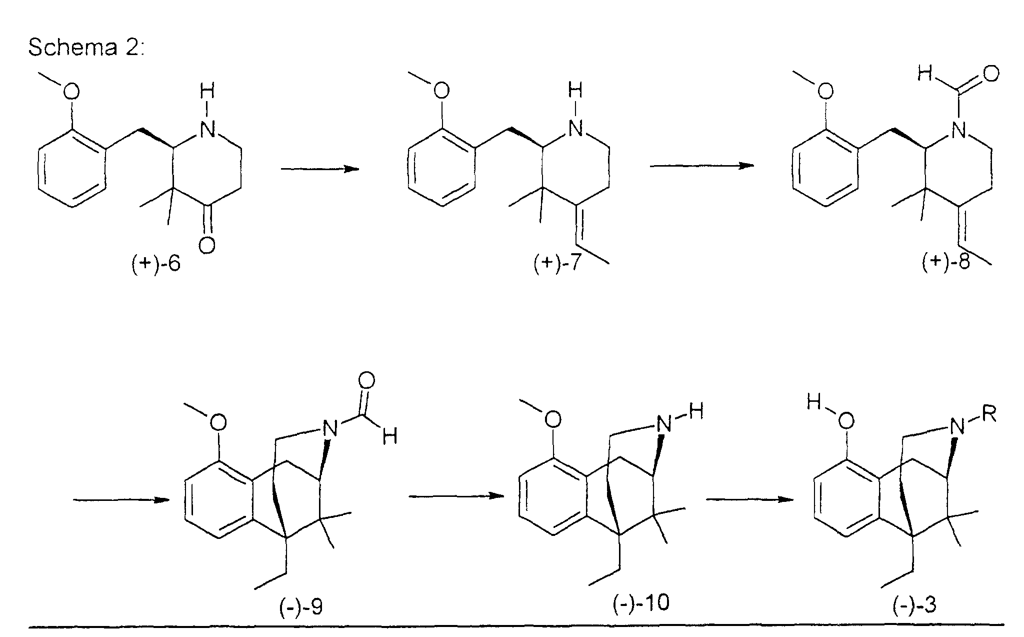

Verbindung **4** wird in Analogie zu dem in der WO 97/06146 beschriebenen Verfahren aus 2-Methoxybenzylcyanid (**11**) und 2-Brompropionsäure (**12**) hergestellt.

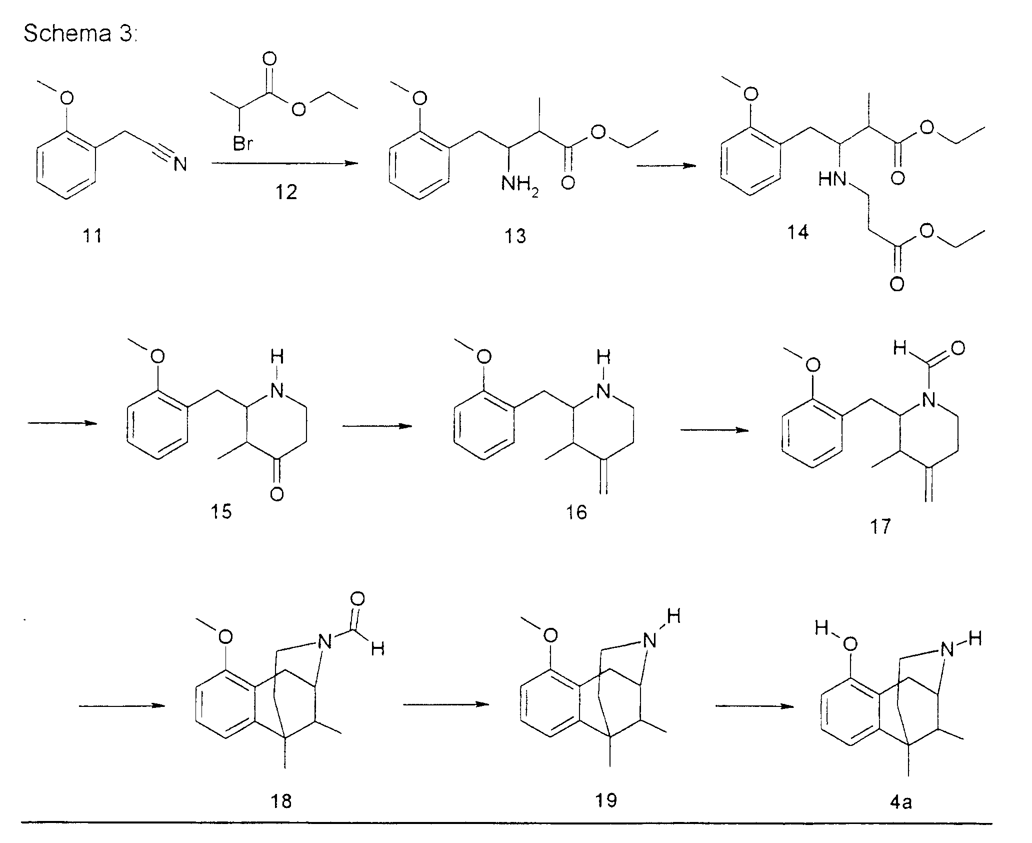

Dazu wird beispielsweise in der ersten Stufe 2-Methoxybenzylcyanid (11 )mit dem 2-Brompropionsäureethylester (12) zum entsprechend substituierten 3-Amino-2-methylbutansäureesterderivat (13) umgesetzt (da es mit Blick auf das angestrebte Endprodukt nicht auf die Alkoholkomponente der Esterpartialstruktur ankommt, kann auch hier jedweder andere C₁-C₈-Alkylester oder auch ein Benzylester eingesetzt werden):

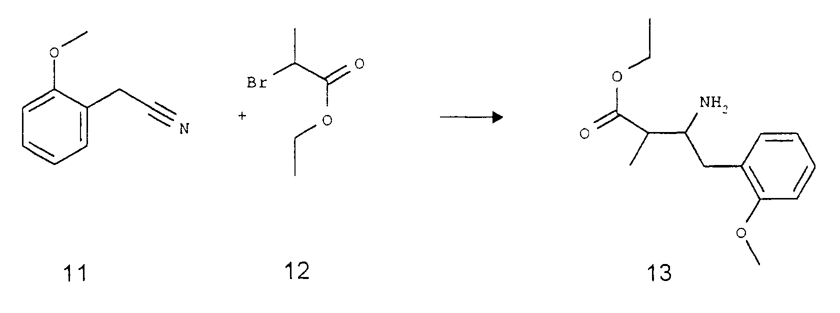

Zur Durchführung dieser Umsetzung vom Typ einer Reformatsky-Reaktion wird ein Alkylhalogensilan - vorzugsweise ein Trialkylchlorsilan, besonders bevorzugt Trimethylchlorsilan - und Zinkpulver in einem unter den gewählten Reaktionsbedingungen inerten Lösungsmittel - vorzugsweise einem Ether oder in einem Halogenkohlenwasserstoff, besonders bevorzugt Dichlormethan - vorgelegt. Nach dem Verdünnen dieser Mischung mit einem polaren - inerten - Lösungsmittel, vorzugsweise einem cyclischen Ether, besonders bevorzugt Tetrahydrofuran - wird die Reaktionsmischung - vorzugsweise auf Rückflußtemperatur - erwärmt und mit einer Mischung des 2-Brompropionsäureethylesters der allgemeinen Formel 3 mit dem o-Methoxybenzylcyanid versetzt und weiter - vorzugsweise auf Rückflußtemperatur - erwärmt. Nach dem Abkühlen und dem Abfiltrieren des Zinkpulvers wird das Reaktionsgemisch mit einem hinsichtlich der Reduktion von Iminofunktionen selektiven Reduktionsmittel - vorzugsweise einem komplexen Alkaliborhydridderivat, besonders bevorzugt Natriumcyanoborhydrid - und anschließend mit einem Alkanol - vorzugsweise einem geradkettigen oder verzweigten C₁-C₄-Alkohol, besonders bevorzugt Ethanol - versetzt. Danach wird mit einer wässerigen Lösung einer basisch reagierenden Verbindung - vorzugsweise mit Ammoniaklösung, besonders bevorzugt mit konzentrierter Ammoniaklösung - versetzt und die organische Phase des Reaktionsgemisches isoliert. Nach dem Trocknen und dem Einengen i.Vak. wird der verbliebene Rückstand in einem inerten Lösungsmittel - vorzugsweise in einem aliphatischen oder aromatischen Kohlenwasserstoff, besonders bevorzugt in Toluol - aufgenommen und mit der wässerigen Lösung einer Säure - vorzugsweise einer Mineralsäure, besonders bevorzugt 2N Salzsäure - extrahiert. Abschließend wird die wässerige Phase mit der wässerigen Lösung einer basisch reagierenden Verbindung - vorzugsweise Ammoniaklösung, besonders bevorzugt mit konzentrierter Ammoniaklösung - alkalisch gestellt und danach mit einem organischen, mit Wasser nicht mischbaren Extraktionsmittel - vorzugsweise mit einem Halogenkohlenwasserstoff, besonders bevorzugt mit Dichlormethan - extrahiert. Der so erhaltene Exrtrakt wird nach dem Trocknen eingeengt und das 3-Amino-2-methylbutansäureesterderivat der allgemeinen Formel 4 isoliert.

In der zweiten Reaktionsstufe wird das so gewonnene 3-Amino-2-methylbutansäureethylesterderivat 13 mit Acrylsäureethylester (da es mit Blick auf das angestrebte Enprodukt nicht auf die Alkoholkomponente der Esterstruktur ankommt, kann auch hier jedweder andere C₁-C₈-Alkylester bzw auch ein Benzylester eingestzt werden) zu dem entsprechenden 3-(2-Ethoxycarbonylethyl)amino-2-methylbutansäureethylesterderivat 14 umgesetzt:

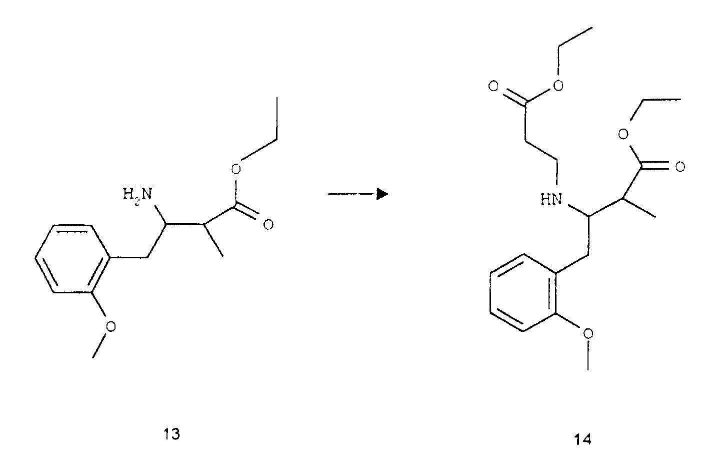

Zur Durchführung dieser Michael-Additionsreaktion wird das 3-Amino-2-methylbutansäureethylesterderivat 13 mit dem Acrylsäureethylester in einem unter den gewählten Reaktionsbedingungen inerten Reaktionsmedium - vorzugsweise in einem geradkettigen oder verzweigten C₁-C₄-Alkanol, besonders bevorzugt Ethanol - gelöst und - vorzugsweise auf Rückflußtemperatur - erwärmt. Nach erfolgter Reaktion wird das Lösungsmittel i.Vak. entfernt und das resultierende 3-(2-Ethoxycarbonylethyl)amino-2-methylbutansäureethylesterderivat 14 isoliert.

In der anschließenden dritten Reaktionsstufe wird das aus der vorangegangenen Reaktionsstufe resultierende 3-(2-Ethoxycarbonylethyl)amino-2-methylbutansäureesterderivat 14 zum entsprechenden Piperidonderivat 15 cyclisiert:

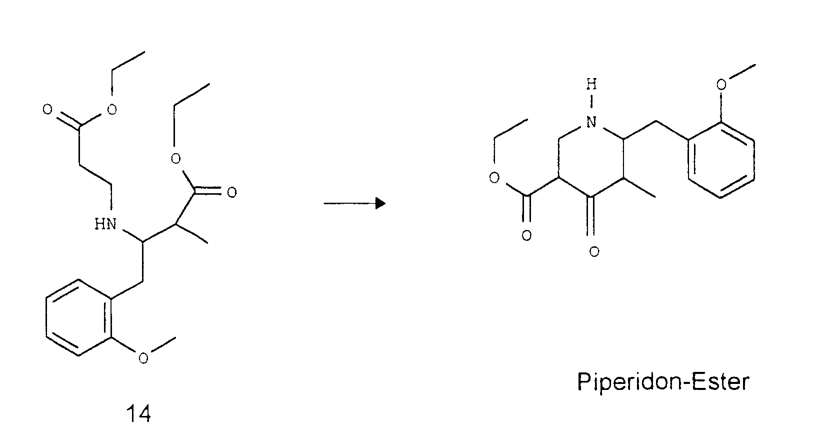

Zur Durchführung des Cyclisierungsschritts vom Typ einer Dieckmann'schen Esterkondensation wird das 3-(2-Ethoxycarbonylethyl)amino-2-methylbutansäureesterderivat 14 in einem unter den Cyclisierungsbedingungen inerten Lösungsmittel - vorzugsweise in einem aliphatischen oder aromatischen Kohlenwasserstoff, besonders bevorzugt in Toluol - gelöst und in Gegenwart einer basisch reagierenden Verbindung, vorzugsweise einem Alkalialkoholat eines verzweigten oder unverzweigten C₁-C₄-Alkohols, besonders bevorzugt Kalium-*tert*.-butylat - auf Rückflußtemperatur erhitzt und die bei diesen Temperaturen flüchtigen Bestandteile der Reaktionsmischung destillativ - beispielsweise im Rahmen einer azeotropen Reaktion - entfernt. Nach beendeter Reaktion, wird das Reaktionsgemisch hydrolysiert und mit der wässerigen Lösung einer sauer reagierenden Verbindung - vorzugsweise mit wässerigen Mineralsäuren, besonders bevorzugt mit konzentrierter Salzsäure - versetzt. Danach wird ein unter diesen Bedingungen inertes, mit Wasser nicht mischbares Extraktionsmittel - bevorzugt ein Dialkylether, besonders bevorzugt Diethylether - zugefügt und mit der wässerigen Lösung einer basisch reagierenden Verbindung, bevorzugt mit wässeriger Ammoniaklösung, besonders bevorzugt mit konzentrierter Ammoniaklösung - versetzt. Nach dem Abtrennen der organischen Phase sowie dem erschöpfenden Extrahieren der wässerigen Phase werden die vereinigten organischen Extrakte nach dem Waschen mit Wasser sowie Trocken i. Vak. eingeengt und den resultierenden Piperidon-Ester isoliert.

Alternativ kann die oben beschriebene Dieckmann-Kondensation auch mittels Titantetrachlorid In einem halogenierten Kohlenwasserstoff - vorzugsweise Dichlormethan - durchgeführt werden [M.N. Deshmukh et al., Synth. Commun. 25 (1995) 177]

In der vierten Reaktionsstufe wird das so erhaltene Piperidonderivat (Piperidon-Ester) unter alkalischen oder sauren Bedingungen zu dem entsprechenden 3-Methyl-4-piperidonderivat verseift und decarboxyliert.

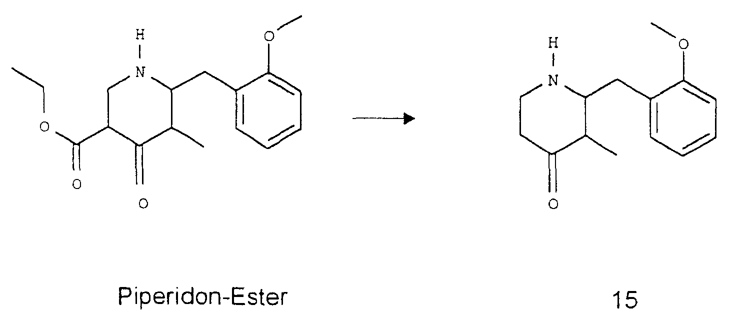

Dazu wird der Piperidon-Ester in einem polaren, wasserhaltigen Lösungsmittel bzw. Lösungsmittelgemisch - bevorzugt in einer Mischung aus einem geradkettigen oder verzweigten C₁-C₄-Alkanol und Wasser, besonders bevorzugt in einem Ethanol/Wasser-Gemisch - mit einer basisch oder sauer reagierenden Verbindung - bevorzugt mit einem Alkalihydroxyd oder einer Mineralsäure, besonders bevorzugt mit Natriumhydroxyd oder im Falle des Einsatze einer Säure beispielsweise in Gegenwart von Salzsäure oder Schwefelsäure - erhitzt; bevorzugt wird auf Rückflußtemperatur erwärmt. Nach erfolgter Verseifung wird das Reaktionsmedium i. Vak. entfernt und der Rückstand in einem für die anschließende Salzbildung geeigneten Lösungsmittel - bevorzugt einem polaren organischen Lösungsmittel, besonders bevorzugt in Aceton - aufgenommen und das entsprechende Säureadditionssalz gefällt.

Die nachfolgende Wittig-Reaktion mit Methyltriphenylphosphoniumbromid führt auf der nächsten Stufe zum entsprechenden 4-Methylen-piperidinderivat 16, das in Form seines Säureadditionssalzes - vorzugsweise in Form eines Hydrohalogenids, besonders bevorzugt in Form seines Hydrochlorids - isoliert werden kann.

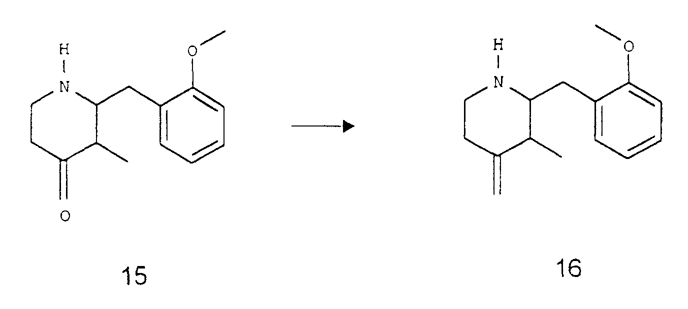

Zur Durchführung der Wittig-Reaktion wird das 3-Methylpiperidonderivat 15 in Form seines Säureadditionssalzes - beispielsweise als Hydrochlorid - in Wasser gelöst und mit einer basisch reagierenden Verbindung oder - vorzugsweise - deren wässerigen Lösung versetzt; besonders bevorzugt wird konzentrierte wässerige Ammoniaklösung eingesetzt.

Die wässerige Phase wird einem organischen, mit Wasser nicht mischbaren Lösungsmittel - bevorzugt mit einem Halogenalkan, besonders bevorzugt mit Dichlormethan - extrahiert. Nach dem Trocknen und dem Einengen i. Vak. wird der Rückstand in einem unter den für die Wittig-Reaktion angewandten Reaktionsbedingungen inerten Reaktionsmedium - bevorzugt in einem cyclischen Ether, besonders bevorzugt in Tetrahydrofuran - aufgenommen und mit einem eine Methylengruppe generierenden Wittig-Reagenz - bevorzugt einem Methyltriphenylphosphoniumhalogenid, besonders bevorzugt mit Methyltriphenylphosphoniumbromid - in Gegenwart einer basisch reagierenden Verbindung, bevorzugt einem Alkalialkoholat, besonders bevorzugt Kalium-*tert*.-butanolat versetzt und - in Abhängigkeit von der Reaktionsfähigkeit der jeweils eingesetzten Edukte - bei einer Temperatur im Bereich von 0 bis 80 °C - bevorzugt in einem Bereich von 20 bis 60 °C und besonders bevorzugt bei ca. 40 °C - umgesetzt. Nach erfolgter Umsetzung wird das Reaktionsgemisch mit Wasser und einem mit Wasser nicht mischbaren organischen Lösungsmittel - bevorzugt mit einem Halogenalkan, besonders bevorzugt Dichlormethan - versetzt und die organische Phase separiert. Nach erschöpfender Extraktion der wässerigen Phase und dem Trocknen der vereinigten Extrakte wird das Extraktionsmittel entfernt, der Rückstand mit einem für die Bildung eines Säureadditionssalzes geeigeneten Lösungsmittels, bevorzugt in einem verzweigten oder unverzweigten C₁-C₄-Alkanol, besonders bevorzugt in Isopropanol, gelöst und mit einer geeigneten Säure, bevorzugt einer Mineralsäure - wie z.B. einer Halogenwasserstoffsäure, besonders bevorzugt mit konzentrierter Salzsäure - versetzt und das auskristallisierte Säureadditionssalz des Wittig-Produktes 16 isoliert.

In der anschließenden Reaktionssstufe erfolgt die Formylierung des Piperidin-Stickstoffs - beispielsweise mit n-Butylformiat - woraus das entsprechende N-Formyl-3-methyl-4-methylen-piperidinderivat resultiert:

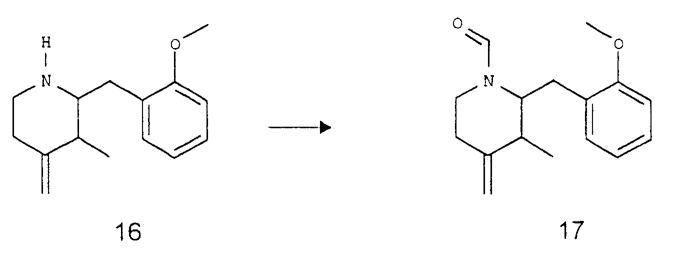

Hierzu wird das Piperidinderivat vom Typ 16, das auf der vorangegangenen Stufe beispielsweise als Hydrohalogenid isoliert werden kann, zunächst in die entsprechende freie Base überführt, indem beispielsweise das Piperidinderivat in Wasser gelöst wird und mit einer basisch reagierenden Verbindung - vorzugsweise mit der wässerigen Lösung einer basisch reagierenden Verbindung und besonders bevorzugt mit konzentrierter Ammoniaklösung - versetzt, das freie Piperidin mit einem organischen Lösungsmittel, bevorzugt mit einem halogenierten Kohlenwasserstoff und besonders bevorzugt mit Dichlormethan, extrahiert. Nach dem Trocknen des Extraktes und dem Abdestillieren des Extraktionsmittels wird die freie Base in einem organischen Lösungsmittel - wie z.B. einem Kohlenwasserstoff, bevorzugt in einem Alkylaromaten, besonders bevorzugt in Toluol - aufgenommen und mit einem Formylierungsmittel - bevorzugt mit einem Alkylformiat, besonders bevorzugt mit n-Butylformiat - zur Reaktion gebracht und das Reaktionsprodukt 17 isoliert.

In der darauf folgenden Cyclisierungsreaktion erfolgt in Gegenwart entsprechend reaktiver Lewis-Säuren - wie beipielsweise Mineralsalsäuren, insbesondere Bromwasserstoffsäure und bevorzugt mit Sulfonsäuren oder mit Aluminium(III)halogeniden, wie z. B. Aluminiumtrichlorid - auf der achten Reaktionsstufe letztendlich der Aufbau des Benzomorphangerüsts vom Typ 18.

Die sich anschließende Reaktionsstufe resultiert in der Abspaltung der Formylgruppe und führt somit zum entsprechenden 4'-Methoxy-5,9-dimethyl-6,7-benzomorphan (19).

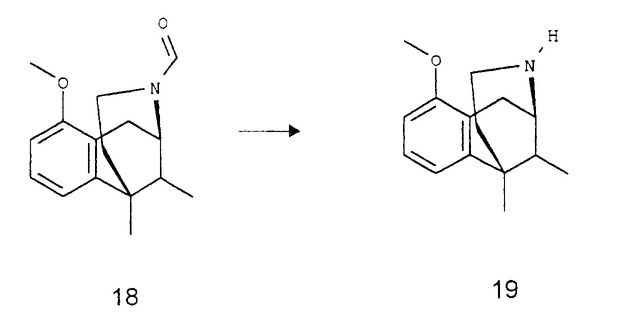

Hierzu wird das Formylbenzomorphan 18 in einem polaren Lösungsmittel - bevorzugt in einem Alkanol, besonders bevorzugt in n-Propanol - gelöst und mit einer sauer reagierenden Verbindung - bevorzugt mit der wässerigen Lösung einer Mineralsäure, besonders bevorzugt mit konzentrierter Salzsäure - versetzt und anschließend erwärmt. Nach erfolgter Abspaltung der Formylgruppe wird die Reaktionsmischung nach dem Einengen mit Wasser versetzt und mit einem mit Wasser nicht mischbaren Lösungsmittel - bevorzugt mit einem Ester einer Carbonsäure, besonders bevorzugt Essigsäureethylester - extrahiert. Die so gereinigte wässerige Phase wird bevorzugt mit konzentrierter Ammoniaklösung basisch gestellt und mit einem organischen Lösungsmittel - bevorzugt mit einem Halogenkohlenwasserstoff, besonders bevorzugt mit Dichlormethan - extrahiert. Nach dem Trocknen und Einengen der vereinigten organischen Extrakte kann auf diese Weise zum Beispiel das entsprechende (-)-4'-Methoxy-5,9-dimethyl-6,7-benzomorphan (19) isoliert werden.

Auf dieser Stufe kann - falls noch nicht geschehen, eine Auftrennung der bis jetzt noch als Mischung vorliegenden Stereoisomere erfolgen. Dabei kann die Isolierung nach den bereits geschilderten bzw. nach an sich aus dem Stand der Technik bekannten Verfahren zur Trennung optischer Isomere erfolgen.

Auf der folgenden Stufe wird das so erhaltene (-)-4'-Methoxy-5,9-dimethyl-6,7-benzomorphan (19) unter sauren Bedingungen - vorzugsweise mit einer Mineralsäure, wie zum Beispiel mit Halogenwasserstoffsäure und besonders bevorzugt mit Bromwasserstoffsäure - einer Etherspaltung unterworfen werden, woraus die entsprechende freie Phenolteilstruktur resultiert.

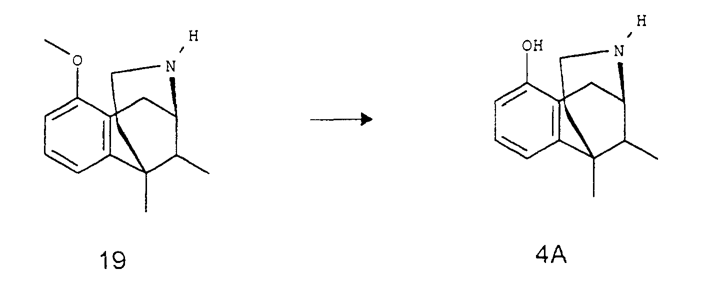

Die Etherspaltung erfolgt dabei unter sauren Bedingungen - wobei sich die Verwendung von Mineralsäuren als vorteilhaft erwiesen hat. Besonders vorteilhaft hat sich u.a. die Verwendung von Bromwasserstoffsäure herausgestellt. Das aus dieser Verseifungsreaktion resultierende Verseifungsprodukt kann auf diese Weise - zum Beispiel - in Form seines Hydrobromids gewonnen werden.

Die Verbindung 5 wird gemäß der in Schema 4 gezeigten Reaktionsfolge hergestellt.

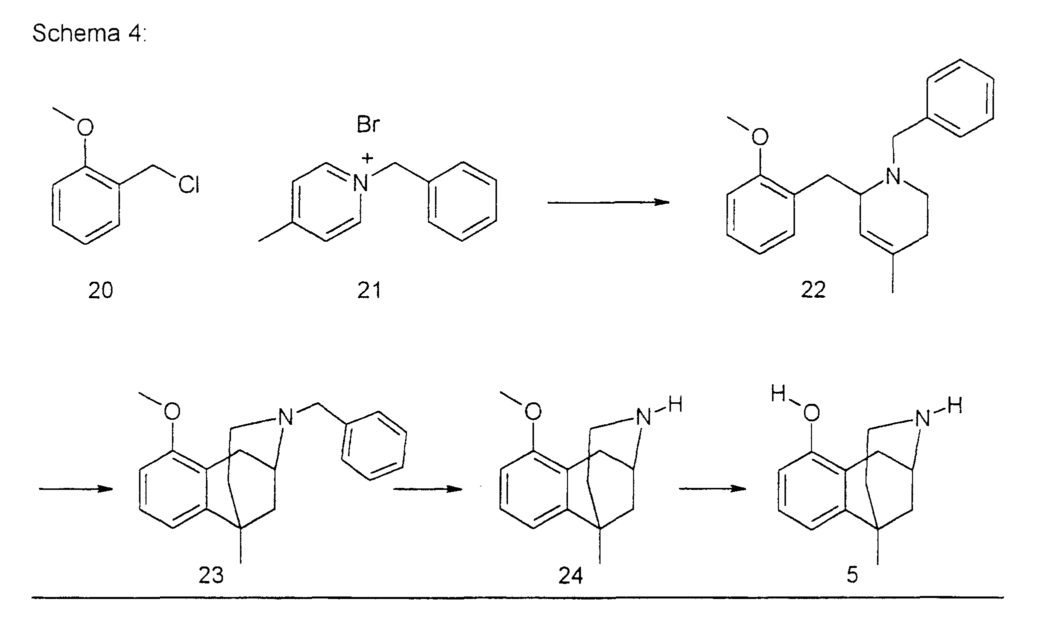

Die Einführung des N-Substituenten erfolgt durch Umsetzung der Schlüsselverbindungen **2a** bis **5a** mit Acylierungsmitteln zu den Zwischenverbindungen **25** und deren anschließender Reduktion oder durch direkte Alkylierung der Schlüsselverbindungen **2a** bis **5a** mit Alkylierungsmitteln oder durch Umsetzung mit Aldehyden zu **26** und anschließender Reduktion. In Schema 5 sind diese Wege beispielhaft für die Schlüsselverbindung (-)-**2a** angegeben.

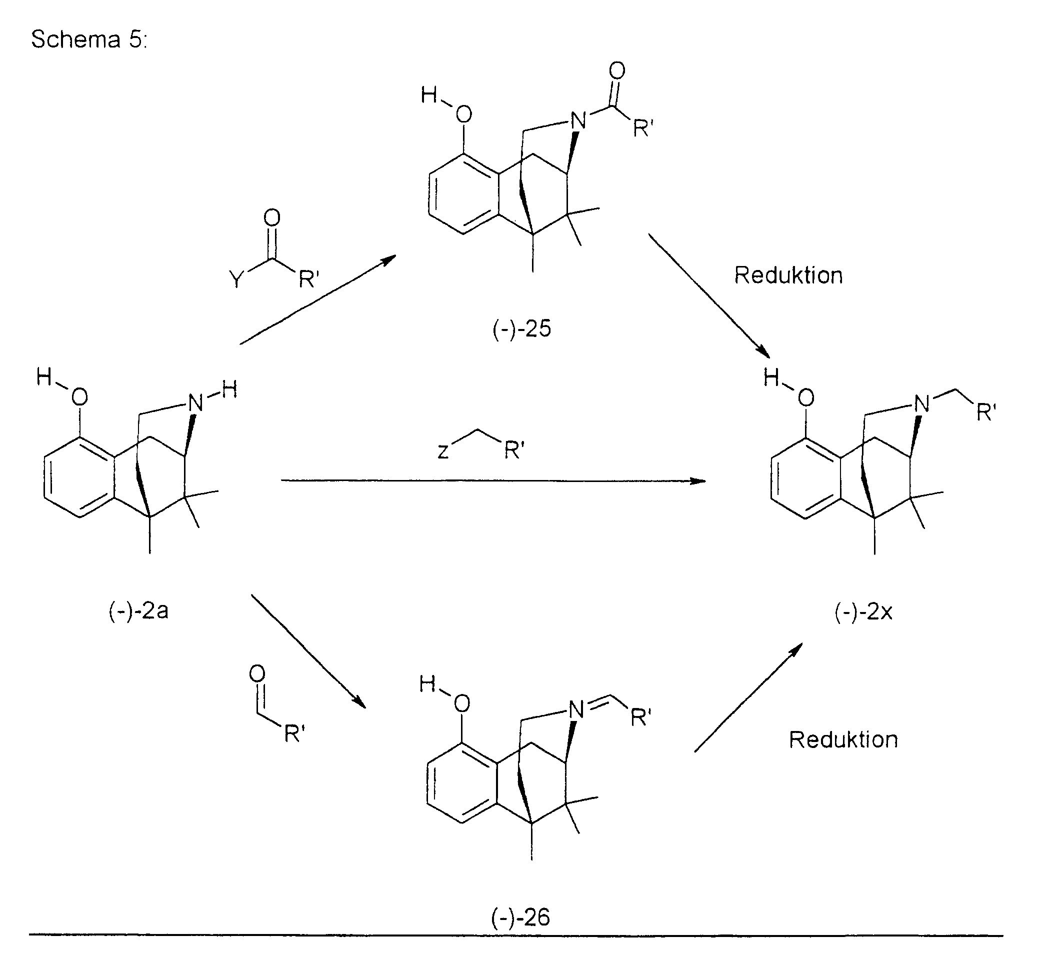

Die erfindungsgemäßen Verbindungen können - nach an sich aus dem Stand der Technik bekannten Methoden - anschließend regioselektiv im Benzomorphanaromaten substituiert werden. Ein Beispiel zur Einführung eines Substituenten R³ gemäß der Allgemeien Formel I ist für Verbindung (-)-**2b** in Schema 6 angegeben.

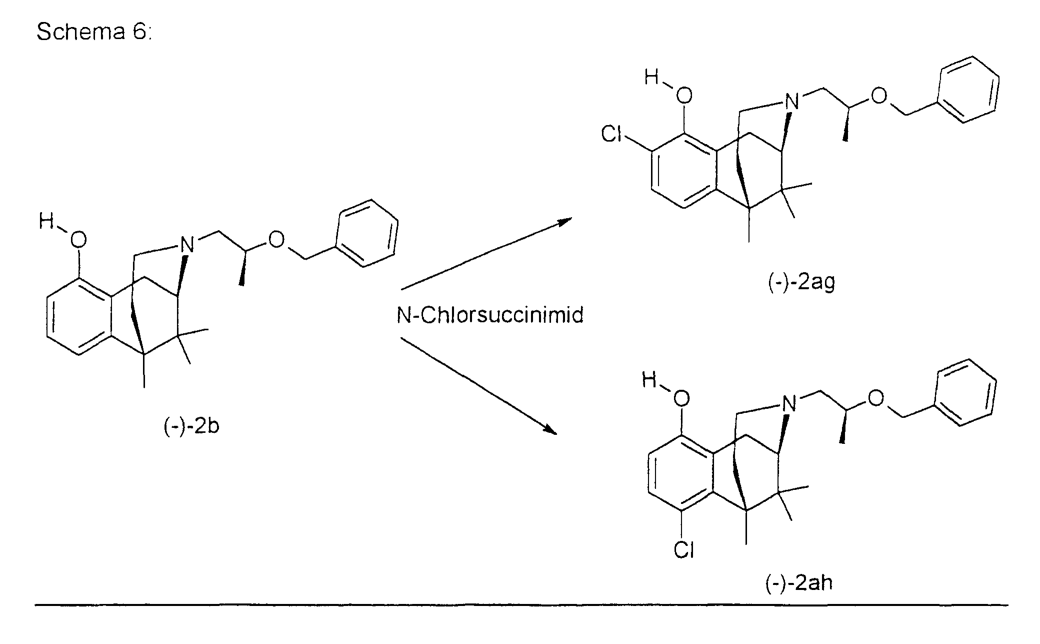

### Biologische Eigenschaften

Es hat sich herausgestellt, daß infolge von Hypoglykämie, Hypoxie, Anoxie und Ischämie auftretende Zellschäden und Funktionsausfälle z.T. auf einer erhöhten synaptischen Aktivität beruhen. Durch eine Reihe von Experimenten konnte gezeigt werden, daß solche hypoglykämischen und hypoxischen Zustände zu einer massiven Depolarisation der betroffenen Zellen führen. Durch diese Depolarisation wird wiederum ein pathogener Anstieg von intracellulären Calcium und in neuronalen Gewebe zusätzlich eine erhöhte Freisetzung von exitatorischen Aminosäuren bewirkt. Der spannungsabhängige Natriumkanal hat eine Schlüsselrolle in dieser Kaskade. So kann durch dessen Blockade die Depolarisation der Zellen verhindert werden, wodurch der Calciumeinstrom durch spannungsabhängige Calciumkanäle und in neuronalen Gewebe zusätzlich auch durch NMDA-Rezeptorkanäle reduziert wird. Weiterhin wird durch den reduzierten Einstrom von Natriumionen in die Zelle verhindert, daß der Calcium/Natrium-Austauscher gegenläufig arbeitet und Calzium in die Zelle hineinfördert. In neuronalen Gewebe verhindert der reduzierte Einstrom von Natriumionen in die Zelle außerdem noch, daß der Glutamat-Transporter gegenläufig arbeitet und Glutamat freisetzt [C.P. Taylor und B.S. Meldrum, TIPS 16 (1995) 309; J. Urenjak und T.P. Obrenovitch Am. Soc. Phar. Exp. Ther. 48 (1996) 21].

Überraschenderweise hat sich nun herausgestellt, daß die erfindungsgemäßen Verbindungen der allgemeinen Formel I im Gegensatz zu den aus den Stand der Technik bekannten Verbindungen [EP-B-0 521422] keine nennenswerte Affinitäten zum NMDA-Rezeptor, besitzen (Kᵢ [³H] MK801: > 10000 nM). Dagegen hat sich vielmehr herausgestellt, daß die erfindungsgemäßen Verbindungen Blocker des spannungsabhängigen Natriumkanals sind. Es handelt sich dabei um Verbindungen, die Batrachotoxin(BTX) mit hoher Affinitat kompetitiv oder nicht-kompetitiv von der Bindungsstelle am Natriumkanal verdrängen. Solche Substanzen zeigen eine "use-dependency" bei der Blockade der Natriumkanäle, d.h. für die Bindung der Substanzen an dem Natriumkanal müssen die Natriumkanäle zunächst aktiviert werden. Die maximal Blockade der Natriumkanäle wird erst nach wiederholter Stimmultion der Natriumkanäle erreicht. Demzufolge binden die Substanzen bevorzugt an Natriumkanäle, die vermehrt aktiviert werden. Dadurch sind die Substanzen in der Lage, bevorzugt in den Körperregionen wirksam zu werden die phatologisch überstimmuliert sind.

Als Testsystem für den Nachweis der Natriumkanal blockierenden Wirkung dient die BTX-Bindung am Natriumkanal [S.W. Postma & W.A. Catteral, Mol. Pharmacol. 25, 219-224 (1984)] sowie patch-clamp Experimente in denen gezeigt werden kann, daß die erfindungsgemäßen Verbindungen den elektrisch stimmulierten Natriumkanal in einer "use-dependent" Weise blockieren [W.A. Catteral, Trends Phamacol. Sci., 8, 57-65 (1987)].

Ferner wurde eine neuroprotektive Wirkung der erfindungsgemäßen Verbindungen durch die Blockade des Veratridin induzierten Glutamat-releases nachgewiesen [S. Villauneva, P. Frenz, Y. Dragnic, F. Orrego, Brain Res. 461, 377-380 (1988)]. Veratridin ist ein Toxin, das den Natriumkanal permanent öffnet. Dadurch kommt es, zu einem erhöhten Einstrom von Natriumionen in die Zelle. Über die oben beschriebene Kaskade führt dieser erhöhte Natriumeinstrom in neuronalem Gewebe zu einem erhöhten Freisetzung von Glutamat. Durch die erfindungsgemäßen Verbindungen läßt sich diese erhöhte Glutamatfreisetzung antagonisieren.

Antikonvulsive Eigenschaften der erfindungsgemäßen Substanzen wurde durch eine protektive Wirkung gegen Krämpfe, die durch einen maximalen Elektoschock in Mäusen ausgelößt wurden, belegt [M.A. Rogawski & R.J. Porter, Pharmacol Rev. 42, 223-286 (1990)] - neuroprotektive Eigenschaften wurden durch eine protektive Wirkung in einem Ratten-MCAO-Modell belegt. [U. Pschorn & A.J. Carter, J Stroke, Cerebrovascular Diseases, 6, 93-99 (1996)].

Ferner wurde beschrieben, daß Natriumkanalblocker zur Therapie der Zyklophrenie (manisch depressive Erkrankung) eingesetzt werden können [J.A. Calabrese, C.Bowden, M.J. Woyshville in : Phsychopharmacology: The fourth generation of progress (Eds.: D.E. Bloom & J. Kupfer) 1099-1111, Raven Press Ltd. New York]. Diese Ergebnisse liefern einen Beleg dafür, daß die 1,2,3,4,5,6-Hexahydro-2,6-methano-3-benzazocin-10-ole der allgemeinen Formel I bei Erkrankungen eingesetzt werden können, derren Ursache auf eine durch Übererregung bedingte Funktionsstörung beruhen. Darunter fallen Erkrankungen wie Arrhythmien, Spasmen, kardiale und Gehirnischämien sowie neurodegenerativen Erkrankungen verschiedener Genese. Beispielsweise kann genannt werden:Status epileptikus, Hypoglykämie, Hypoxie, Anoxie, Gehirntrauma, Gehirnoedem, Gehirn-Schlaganfall, perinatale Asphyxie, amylotrope laterale Sklerose, Huntington's Disease, Morbus Alzheimer, Morbus Parkinson, Zyklophrenie, Hypotonie, Herzinfakt, Herzrhythmusstörungen, Angina pectoris, Schmerz, Anaesthesie und Lokalanaesthesie.

Als ganz besonders wirksam haben sich in diesem Zusammenhang folgende Verbindungen herausgestellt:
(-)-(1R,2"S)-2-(2"-Benzyloxy)propyl-4'-hydroxy-5,9,9-trimethyl-6,7-benzomorphan und
(-)-(1 R,2"S)-2-[2"-(2"',6"'-Difluorobenzyl)oxy]propyl-4'-hydroxy-5,9,9-trimethyl-6,7-benzomorphan .

Die erfindungsgemäßen Verbindungen sind ausgehend von aus dem Stand der Technik bekannten Verbindungen u.a. nach den in den folgenden Beispielen beschriebenen Verfahren herstellbar.

Insbesondere betrifft die vorliegende Erfindungen u.a. folgendes Verfahren zur Herstellung von Norbenzomorphanen der allgemeinen Formel 5, dadurch gekennzeichnet, daß man
a) o-Methoxychlorbenzylchlorid (20) mit dem Benzylpyridiniumbromid 21 zum Tetrahydropyridin 22 umsetzt 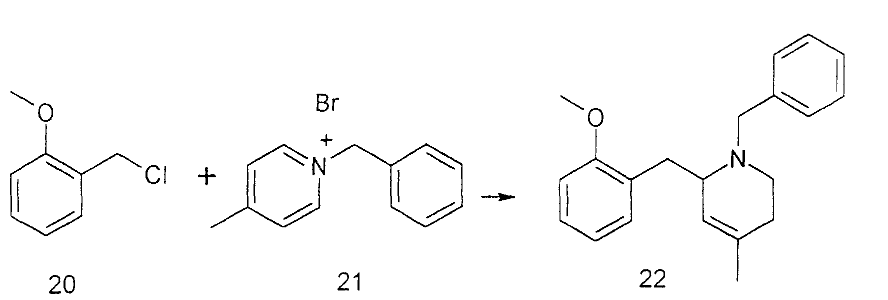 und
b) das Tetrahydropyridinderivat 22 zum N-Benzylbenzomorphanderivat 23 umlagert 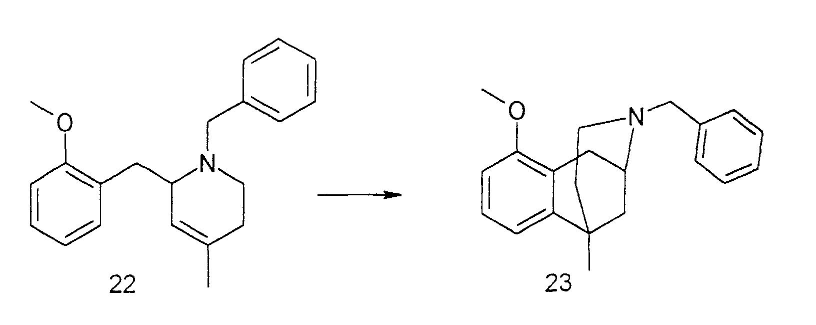 und
c) den Aminostickstoff zum Methoxybenzomorphanderivat 24 debenzyliert 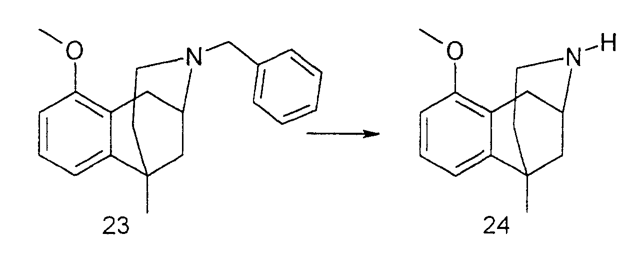 und
d) den Phenolether 24 spaltet und das Benzomorphanderivat 5 isoliert

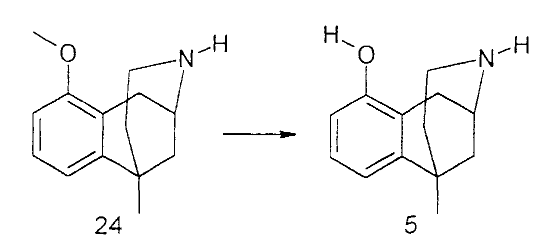

Daneben betrifft die viorliegende Erfindung ein Verfahren zur Herstellung von Norbenzomorphanen der allgemeinen Formel 1 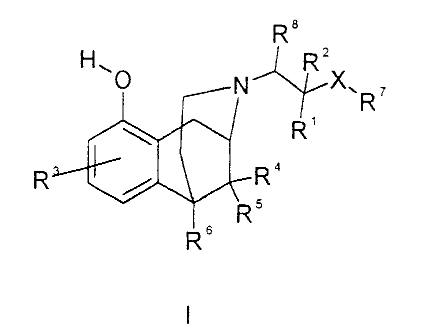 in dem man
a) ein Benzylcyanid der allgemeinen Formel 32 in der R₃₀ eine C₁-C₄-Alkylgruppe bedeutet, mit einem Halogencarbonsäurearbonsäureester der allgemeinen Formel 33, in der R₄₀ C₁-C₈ -Alkyl oder Benzyl bedeutet, in Gegenwart eines Alkylhalogensilans - vorzugsweise einem Trialkylchlorsilan und besonders bevorzugt Trimethylchlorsilan - und Zinkpulver in einem inerten Lösungsmittel - bevorzugt in einem Ether oder in einem Halogenkohlenwasserstoff und besondersbevorzugt mit Dichlormethan und in Gegenwart eines hinsichtlich der Reduktion von Iminofunktionen selektiven Reduktionsmittels - vorzugsweise in Gegenwart eines Alkyliborhydridderivats und besonders bevorzugt in Gegenwart von Natriumcyanoborhydrid - den Bedingungen einer Reformatsky-Reaktion unterwirft und das resultierende Carbonsäuresäureeesterderivat der allgemeinen Formel 34 isoliert 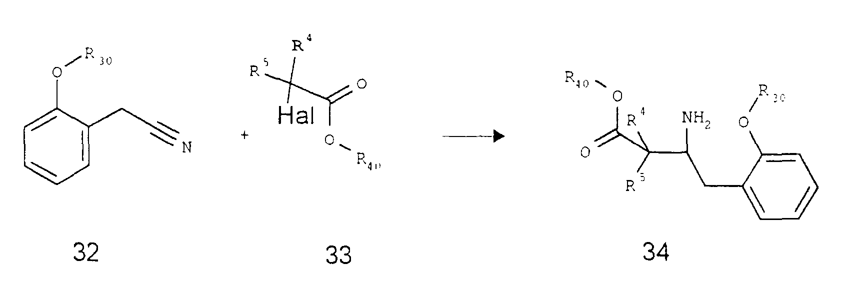 und
b) das Carbonsäureesterderivat der allgemeinen Formel 34 mit einem Acrylsäureester, in dem die Alkoholkomponente R₅₀ die Bedeutung einer C₁-C₈-Alkylgruppe oder einer Benzylgruppe hat, den Bedingungen einer Michael-Additionsreaktion in einem unter den gewählten Reaktionsbedingungen inerten Lösungsmittel - vorzugsweise in einem Alkanol und besonders bevorzugt in Ethanol - unterwirft und das resultierende Michael-Additionsprodukt der allgemeinen Formel 35 isoliert 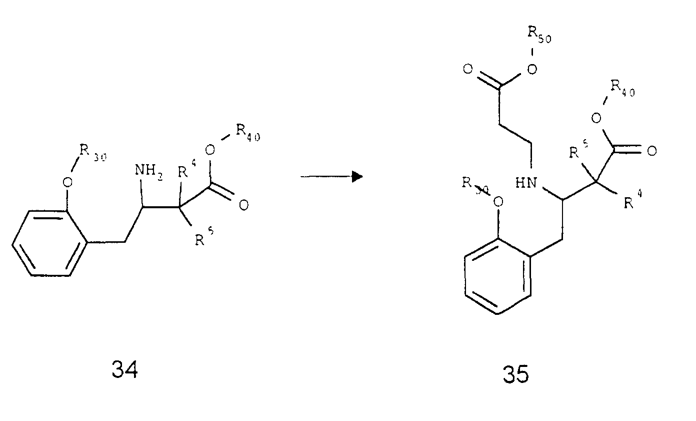 und
c) das so hergestellte Carbonsäurediesterderivat der allgemeinen Formel 35 in einem inerten Lösungsmittel - bevorzugt in einem aliphatischen oder aromatischen Kohlenwasserstoff und besonders bevorzugt in Toluol - in Gegenwart einer basisch reagierenden Verbindung - vorzugsweise in Gegenwart eines Alkalialkoholates eine verzweigten oder unverzweigten C₁-C₄-Alkanols und besonders bevorzugt in Gegenwart von Kalium-*tert*.-butanolat - den Bedingungen einer Dieckmann'schen Esterkondensation unterwirft und das resultierende Piperidonderivat der allgemeinen Formel 36 isoliert 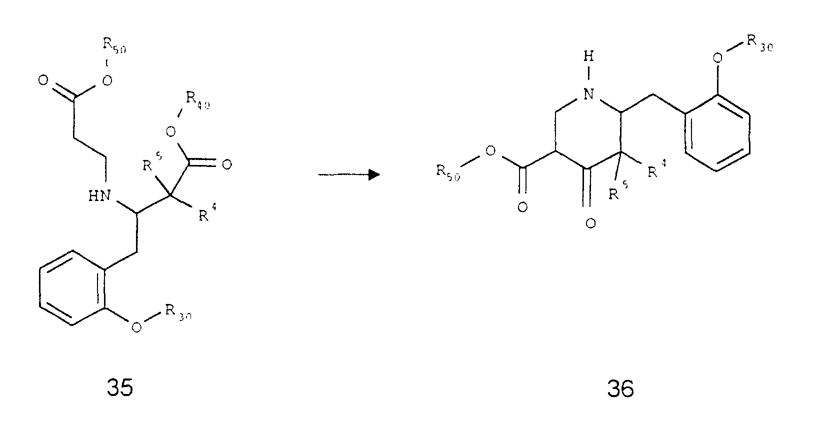 und
d) das Piperidonderivat 36 unter sauren oder alkalischen Bedingungen - bevorzugt in Gegenwart eines Alkalihydroxyds oder einer Mineralsäure und besonders bevorzugt in Gegenwart von Natriumhydroxyd - in einem polaren Lösungsmittel oder Lösungsmittelgemisch - bevorzugt in einer Mischung aus einem geradkettigen oder verzweigten C₁-C₄-Alkanol und Wasser und besonders bevorzugt in einem Ethanol/Wasser-Gemisch - unter Erhitzen zum entsprechenden Piperidonesterderivat der allgemeinen Formel 37 verseift und decarboxyliert, isoliert und gegebenenfalls mit einer Säure das entsprechende Säureadditionssalz herstellt und isoliert 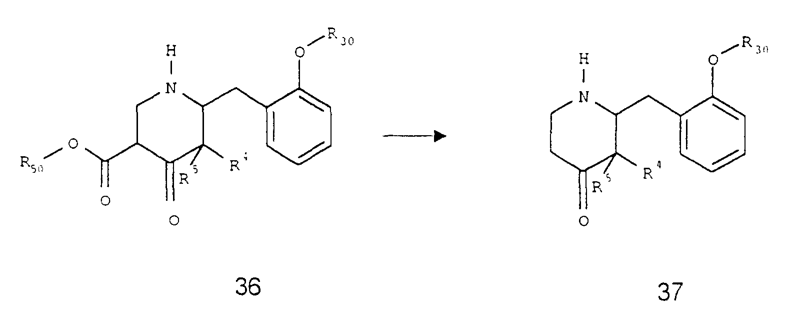 und
e) gewünschtenfalls das so erhaltene Stereoisomerengemisch gegebenenfalls nach Freisetzung der jeweiligen enantiomeren freien Basen in einem - hinsichtlich der Enantiomerentrennung - inerten Reaktionsmedium löst, mit einem geeigneten Stereoisomeren einer zur Salzbildung mit einem Stereoisomeren der Enantiomerenmischung geeigneten organischen Säure versetzt, das gewünschte Stereoisomere in Form seines Additionssalzes mit der optisch aktiven Säure isoliert. 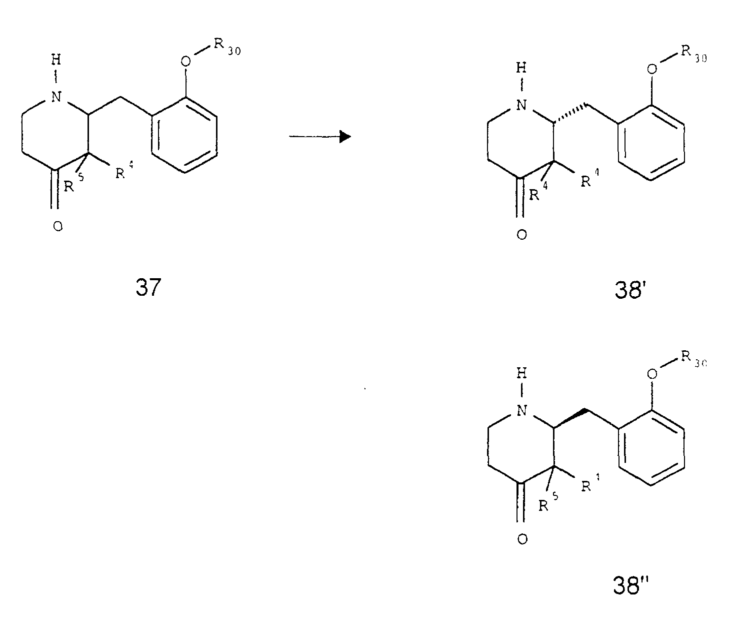 und
f) das so erhaltene reine Stereoisomere 38' bzw 38" oder das Isomerengemisch 38 nach dem Freisetzen aus dem enantiomerenreinen Säureadditionssalz in einem inerten Lösungsmittel mit einem eine CH₂= bzw.CH₃-CH= Gruppierung generierenden Wittig-Reagenz vorzugsweise mit einem Ethyltriphenylphosponiumhalogenid oder mit einem Methyltriphenylphosponiumhalogenid besonders bevorzug mit Methyltriphenylphosponiumbromid bzw. Ethyltriphenylphosponiumbromid in Gegenwart einer basischen reagierenden Verbindung - bevorzugt in Gegenwart eine Alkalialkoholats, besonders bevorzugt Kaliu-tert.-butanolat - in einem inerten Reaktionsmedium - bevorzugt in einem cyclischen Ether, besonders bevorzugt in Tetrahydrofuran - der Wittig-Reaktion unterwirft und das Reaktionsprodukt vom Typ 39 oder das entsprechende Strereoisomere gegebenenfalls in Form seines Säureadditionsalzes isoliert 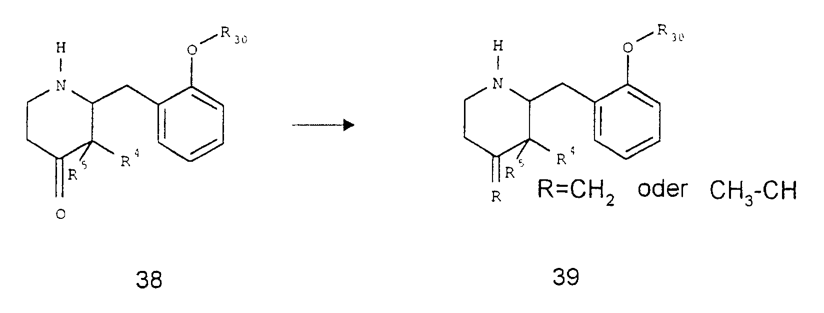 und
g) das aus der Wittig-Reaktion hervorgegangene Alken 39 gegebenenfalls zunächst aus seinem Säureadditionssalz freisetzt und die freie Base vom Typ 39 in einem organischen Lösungsmittel - bevorzugt in einem halogenierten Kohlenwasserstoff und besonders bevorzugt in Dichlormethan - löst und mit einem Formylierungsmittel - bevorzugt n-Butylformiat - einer Formylierungsreaktion am Piperidinstickstoff unterwirft und das Reaktionsprodukt vom Typ 40 oder das entsprechende Stereoisomere isoliert 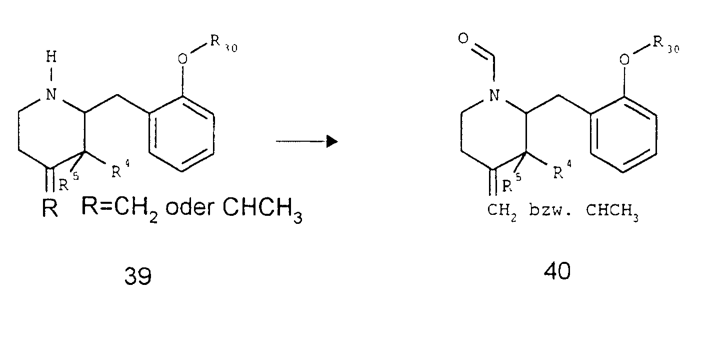 und
h) die so gewonnene Formylverbindung 40 - bzw. das entsprechende Stereoisomere - mit einer Mineralsäure oder mit einer Lewis-Säure - vorzugsweise Aluminium(III)chlorid - in einem inerten Lösungsmittel - vorzugsweise in einem halogenierten Kohlenwasserstoff und besonders bevorzugt in Dichlormethan - löst, umsetzt und das aus dieser Umsetzung resultierende Cyclisierungsprodukt vom Typ 41 isoliert 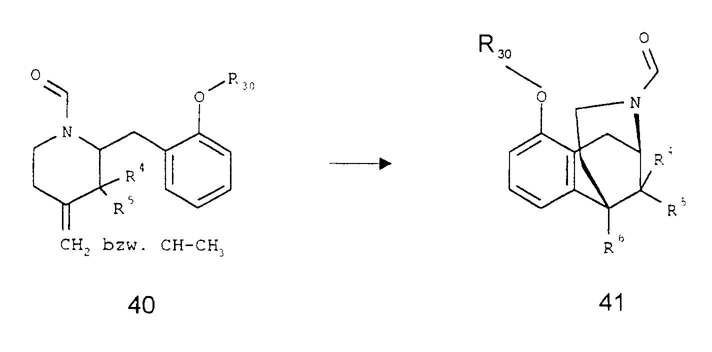 und
i) das aus der Cyclisierungsreaktion resultierende Benzomorphanderivat in einem polaren Lösungsmittel - vorzugsweise in einem C₁-C₄-Alkanol, besonders bevorzugt in n-Propanol - löst und mit einer sauer reagierenden Verbindung - bevorzugt mit der wässerigen Lösung einer Mineralsäure und besonders bevorzugt mit konzentrierter Salzsäure - zur Reaktion bringt und das aus dieser Umsetzung resultierende deformylierte Norbenzomorphan vom Typ 42 gegebenenfalls in Form seines Säureadditionssalzes isoliert und 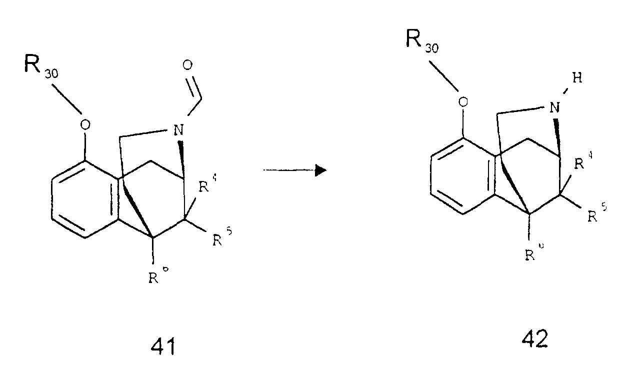
j) falls noch keine Trennung der Stereoisomeren erfolgte auf dieser Stufe die Stereoisomeren auf ans sich bekannte Weise trennt und nach Freisetzung der freien Benzomorphan-Base den Phenolether mit einer sauer reagierenden Verbindung - vorzugsweise mit einer Mineralsäure und besonders bevorzugt mit Bromwasserstoffsäure - spaltet und das Spaltprodukt vom Typ 43 isoliert 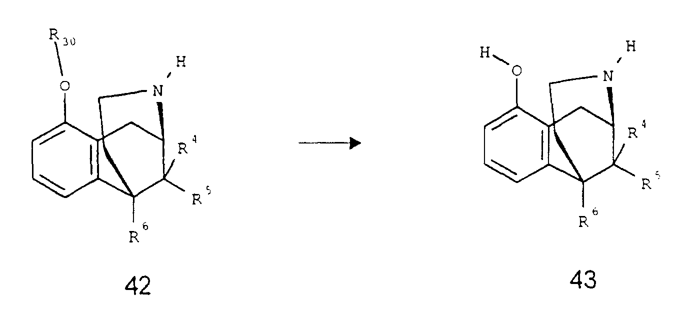
k) das Spaltprodukt 43 mit einer Verbindung vom Typ Z-CHR₈-R' umsetzt in der Z eine durch einen sekundären Aminstickstoff substituierbare Austrittsgruppe bedeutet - bevorzugt Halogen wie Chlor, Brom, lod oder ein organisches Sulfonat, bevorzugt Trifluormethansulfonat und R' die Bedeutung -CR¹R²XR⁷ hat, 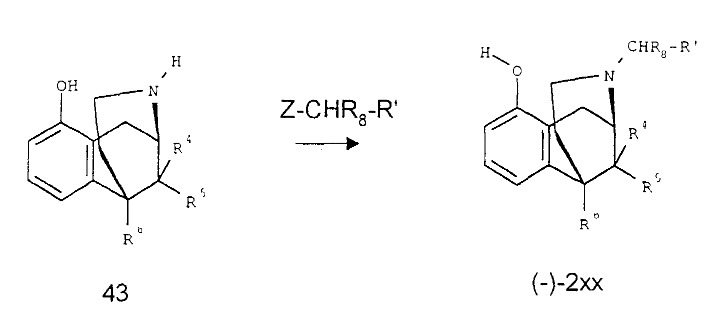 oder
   mit einer Verbindung vom Typ YC(O)R' umsetzt, in der Ydurch einen sekundären Aminstickstoff substituierbare Austrittsgruppe bedeutet - bevorzugt Halogen wie Chlor, Brom, lod oder ein organisches Sulfonat, bevorzugt Trifluormethansulfonat und R' die Bedeutung -CR¹R²XR⁷ hat, und anschließend die Carbonylverbindung zur Verbindung (-)-2xx ― wie oben dargestellt - reduziert 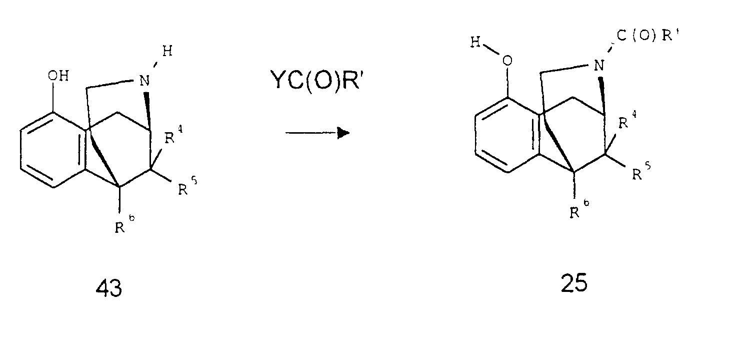 oder
   mit einem Aldehyd der allgemeinen Formel HC(O)-R' umsetzt, in der R' die Bedeutung -CR¹R²XR⁷ hat, und die resultierende Schiff'sche Base 26 zur Verbindung (-)-2xx - wie oben dargestellt - reduziert 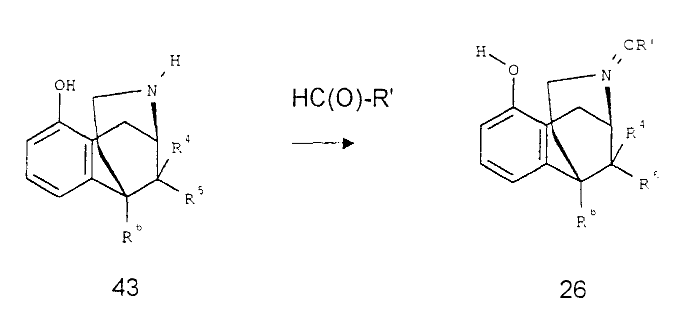 und
I) gegebenenfalls im Rahmen einer elektrophilen Substitution den Substitueneten R³ einführt

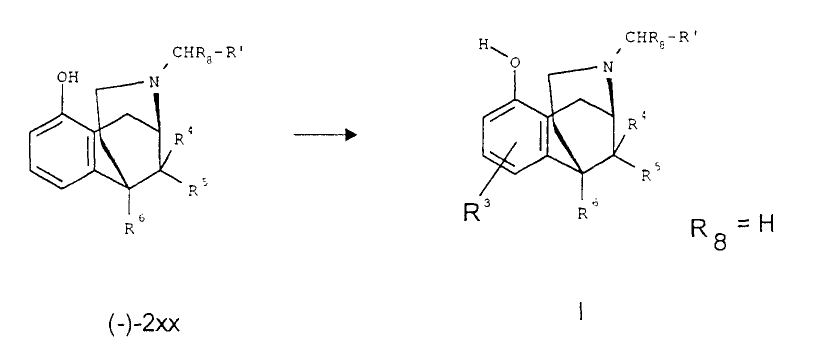

Verschiedenartige, andere Ausgestaltungen der Verfahren werden für den Fachmann aus der vorliegenden Beschreibung ersichtlich. Es wird jedoch ausdrücklich darauf hingewiesen, daß diese Beispiele und die diesen zugeordnete Beschreibung lediglich zum Zweck der Erläuterung vorgesehen und nicht als Einschränkung der Erfindung anzusehen sind.

Daneben wird auf den Inhalt der Deutschen Patentanmeldung Nr. 197 40 110.4, deren Priorität die vorliegende Anmeldung beansprucht, ausdrücklich Bezug genommen.

### Beispiele

### Beispiel 1: 3-Amino-4-(2-methoxyphenyl)-2-methylbutansäureethylester (13)

150 g Zink in 1,5 L absolutem Dichlormethan werden unter Stickstoff mit 15 mL Trimethylchlorsilan versetzt und 30 min. bei Raumtemperatur gerührt. Anschließend werden 900 mL absolutes Tetrahydrofuran (THF) hinzugefügt und auf 42 °C erwärmt. Zu dieser Vorlage läßt man eine Mischung aus 147 g (1,0 mol) 2-Methoxybenzylcyanid (**11**) und 362 g (2,0 mol) 2-Brompropionsäureethylester in 100 mL THF zutropfen und erwärmt anschließend noch 2 h unter Rückfluß. Man läßt abkühlen, dekantiert vom überschüssigem Zink ab und versetzt nach Kühlung auf ca. 5 °C mit 70 g (1,8 mol) Natriumborhydrid. Anschließend werden 250 mL Ethanol zugetropft (Gasentwicklung). Man läßt 3 h bei 5 °C nachreagieren, versetzt mit langsam mit 1I 2 n Salzsäure, trennt die Phasen und extrahiert die wässerige Phase zweimal mit je 200 mL Dichlormethan. Das Lösungsmittel der vereinigten organischen Phase wird im Vakuum abgezogen, der Rückstand mit Eis und Toluol versetzt und mit konz. Ammoniak alkalisch gestellt. Die Phasen werden getrennt und die wäßrige Phase noch zweimal mit je 800 mL Toluol extrahiert. Die vereinigten organischen Phasen werden über Magnesiumsulfat getrocknet, das Lösungsmittel im Vakuum abgezogen. Ausbeute 149 g (61 %) Öl.

### Beispiel 2: 3-(2-Etoxycarbonylethyl)amino-4-(2-methoxyphenyl)-2-methylbutansäureethylester (14)

148 g (0,6 mol) 3-Amino-4-(2-methoxyphenyl)-2-methylbutansäureethylester (**13**) und 119 g (1,2 mol) Acrylsäureethylester werden in 250 mL abs. Ethanol gelöst und 6 h unter Rückfluß erhitzt. Anschließend wird der Ansatz im Vakuum eingeengt. Der Rückstand wird nochmals in 300 mL Toluol aufgenommen und erneut im Vakuum eingeengt. Man isoliert 210 g (100%) des gewünschte Produkts als Öl.

### Beispiel 3: 2-(2-Methoxyphenyl)methyl-3-methyl-4-piperidon (15)

210 g (0,6 mol) 3-(2-Etoxycarbonylethyl)amino-4-(2-methoxyphenyl)-2-methylbutansäureethylester (**14**) werden in 3 l Toluol gelöst und zunächst ca. 100 mL eines Lösungsmittef/Wassergemisches abgeschleppt. Man läßt auf ca. 70 °C abkühlen, versetzt mit 80 g (0.7 mol) Kalium-*tert*.-butylat und erwärmt 30 min. auf 105 °C, wobei das sich bildende Ethanol abdestilliert wird. Anschließend läßt man abkühlen und zieht das Lösungsmittel im Vakuum ab. Der Rückstand wird mit 400 mL Ethanol und 200 mL 40%ige Natronlauge versetzt und 3 h unter Rückfluß gekocht. Man zieht den Alkohol im Vakuum ab und extrahiert die wässerige Phase dreimal mit je 400 mL Diethylether (Ether). Die vereinigten organischen Phasen werden über Magnesiumsulfat getrocknet, das Lösungsmittel im Vakuum abgezogen. Ausbeute 109 g (78%) Öl.

### Beispiel 4: (+)-4-Ethylen-2-(2-methoxyphenyl)methyl-3,3-dimethyl-piperidin ((+)-7)

74,2 g (200 mmol) Ethyl-triphenylphosphoniumbromid werden in 200 mL absolutem Tetrahydrofuran suspendiert und unter Stickstoff mit 80 mL einer 2,5 n Lösung von n-Butyl-Lithium in Hexan versetzt. Man läßt 30 min bei 30 °C rühren, und versetzt anschließend mit einer Lösung von 23 g (93 mmol) des Piperidons **(+)-6** in 100 mL THF. Man läßt 12 h bei Raumtemperatur nachreagieren, fügt 100 mL Wasser hinzu und zieht das THF im Vakuum ab. Der Rückstand wird dreimal mit je 200 mL Essigester extrahiert und die vereinigten organischen Extrakte werden noch einmal mit 50 mL Wasser gewaschen, über Magnesiumsulfat getrocknet und das Lösungsmittel im Vakuum abgezogen. Der Rückstand wird über eine Flashsäule gereinigt (300 mL Kieselgel; 4 l Cyclohexan/ Essigester 3:1). Man isoliert das gewünschte Produkt als Öl in einer Ausbeute von 17,4 g (72%).

Analog zu Beispiel 4 wurde dargestellt:
2-(2-Methoxyphenyl)methyl-3-methyl-4-methylen-piperidin (**16**)

Man verwendet 20, 1 g (56 mmol) Methyl-triphenylphosphoniumbromid, 6,3 g (56 mmol) Kalium-tert.-butylat und 11 g (47 mmol) 2-(2-Methoxyphenyl)methyl-3-methyl-4-piperidon (**15**) in 200 mL abs. THF. Das Produkt wird als Oxalat aus Aceton kristallisiert. Ausbeute: 13,1 g (87%); Schmp.: 145 °C.

### Beispiel 5: (+)-4-Ethylen-N-formyl-2-(2-methoxyphenyl)methyl-3,3-dimethyl-piperidin ((+)-8)

3,5 g (12,6 mmol) (+)-4-Ethylen-2-(2-methoxyphenyl)methyl-3,3-dimethyl-piperidin (**7**) werden mit 20 mL n-Butylformiat 4 h bei 80 °C gerührt. Anschießend wird im Vakuum eingedampft. Es verbleiben 3,6 g (100%) des gewünschten Produkts als Öl.

Analog zu Beispiel 5 wurde dargestellt:
N-Formyl-2-(2-methoxyphenyl)methyl-3-methyl-4-methylen-piperidin (**17**)

8 g (34 mmol) 2-(2-Methoxyphenyl)methyl-3-methyl-4-methylen-piperidin (**16**) als Base und 30 mL n-Butylformiat wurden eingesetzt. Ausbeute: 9,1 g (100%) als Öl.

### Beispiel 6: N-Benzyl-2-(2-methoxyphenyl)methyl-4-metyl-piperi-3-den (22)

6,0 g (250 mmol) Magnesiumspähne und etwas Jod werden in 150 mL Ether vorgelegt. Hierzu werden 31,32g (200 mmol) 2-Methoxybenzylchlorid in 50 mL Ether so zugetropft, daß die Mischung leicht siedet. Man läßt 1 h nachreagieren. Das so erhaltene Grignardreagenz wird anschließend zu einer auf -10 °C gekühlten Suspension von 52,8 g (200 mmol) N-Benzyl-4-methyl-pyridiniumbromid in 100 mL Ether unter Stickstoff schnell zugetropft. Man läßt 2,5 h nachreagieren. Anschließend gibt man den gesamten Reaktionsansatz zu 200 mL einer 10%igen Ammoniumchloridlösung. Die organische Phase wird abgetrennt und die wäßrige Phase noch zweimal mit je 100 mL Ether extrahiert. Die vereinigten organischen Phasen werden über Magnesiumsulfat getrocknet und das Lösungsmittel im Vakuum abgezogen. Um Oxidationen zu vermeiden belüftet man den Rotationsverdampfer mit Stickstoff. Der Rückstand wird sofort in 250 mL Methanol gelöst und 9,5 g (250 mmol) Natriumbohrhydrid und 20 mL 2 N Natronlauge hinzugegeben. Man läßt über Nacht bei RT rühren und engt im Vakuum ein. Der wäßrige Rückstand wird zweimal mit je 150 mL Ether extrahiert und die vereinigte organische Phase im Vakuum eingeengt. Man nimmt den Rückstand in Essigester auf und extrahiert fünfmal mit je 150 mL 2 N Salzsäure. Die vereinigte wäßrige Phase wird anschließend wieder mit Natronlauge alkalisch gestellt und zweimal mit je 200 mL Essigester extrahiert. Die vereinigten organischen Phasen werden über Magnesiumsulfat (MgSO₄) getrocknet und das Lösungsmittel im Vakuum abgezogen. Der Rückstand wird durch eine Filtration über 200 mL Kieselgel (Elutionsmittel: Ether) gereinigt. Man erhält 31 g (51%) des gewünschten Produkts als Öl.

### Beispiel 7: (-)-5-Ethyl-2-formyl-4'-methoxy-9,9-dimethyl-6,7-benzomorphan ((-)-9)

3,6 g (12,6 mmol) (+)-4-Ethylen-N-formyl-2-(2-methoxyphenyl)methyl-3,3-dimethyl-piperidin (**8**) werden mit 35 mL Methansulfonsäure versetzt und 3 h bei 80 °C gerührt. Man läßt abkühlen und gibt die Reaktionsmischung auf 50 g Eis, neutralisiert mit Ammoniak und extrahiert zweimal mit je 100 mL Essigester. Die vereinigten organischen Extrakte werden noch einmal mit 50 mL Wasser gewaschen, über MgSO₄ getrocknet und das Lösungsmittel im Vakuum abgezogen. Der Rückstand wird über eine Flashsäule gereinigt (50 mL Kieselgel; 750 mL Cyclohexan/ Essigester 3:1 ). Man isoliert das gewünschte Produkt als Öl in einer Ausbeute von 2,1 g (58%).

Analog zu Beispiel 7 werden dargestellt:
N-Formyl-4'-methoxy-5,9-dimethyl-6,7-benzomorphan (**18**)

Man verwendet 5,0 g (19 mmol) N-Formyl-2-(2-methoxyphenyl)methyl-3-methyl-4-methylen-piperidin (**17**) und 30 mL Methansulfonsäure. Man erhält 4,8 g (96%) des gewünschten Produkts (Öl) als eine Mischung aus 90% α-Epimer und 10 % β-Epimer.

### N-Benzyl-4'-methoxy-9-methyl-6,7-benzomorphan-oxalat (23OX)

Man verwendet 31 g (100 mmol) N-Benzyl-2-(2-methoxyphenyl)methyl-4-metyl-piperi-3-den (**22**) und 100 mL Methansulfonsäure. Der Rückstand wird in 100 mL Methanol gelöst und mit 60 g Aktivkohle kurz aufgekocht und heiß über Kieselgel abgesaugt. Man zieht das Lösungsmittel im Vakuum ab, löst den Rückstand in Ether und fällt mit Oxalsäure das Oxalat. Man erhält 30 g (75%); Schmp.: 152 °C **(MK 1-11)**.

### Beispiel 8: 4'-Methoxy-9-methyl-6,7-benzomorphan-oxalat (24OX)

Man löst 30 g 75 mmol) N-Benzyl-4'-methoxy-9-methyl-6,7-benzomorphan-oxalat (**23**OX) in 600 mL Methanol und hydriert bei 60 °C und 5 bar an 3 g Pd/Kohle (10%). Man kühlt auf 5 °C und saugt das ausgefallene Produkt ab. Man erhält 17 g (56%), Schmp.: 250 °C **(MK 1-15)**.

### Beispiel 9: (-)-5-Ethyl-4'-methoxy-9,9-dimethyl-6,7-benzomorphan ((-)-10)

2,0 g (6,9 mmol) (-)-5-Ethyl-2-formyl-4'-methoxy-9,9-dimethyl-6,7-benzomorphan (**9**) werden in 30 mL n-Propanol gelöst und mit 10 mL konz. Salzsäure 2h in der Mikrowelle bei 300 Watt erhitzt. Anschließend wird im Vakuum das Lösungsmittel abgezogen, der Rückstand mit 15 mL Eiswasser versetzt und mit zweimal 20 mL Essigester extrahiert (wird verworfen). Die wässerige Phase wird mit konz. Ammoniak neutralisiert und dreimal mit je 20 mL Essigester extrahiert. Die vereinigten organischen Extrakte werden über MgSO₄ getrocknet und das Lösungsmittel im Vakuum abgezogen. Der Rückstand wurde in Aceton gelöst und mit etherischer Salzsäure das Hydrochlorid gefällt. Ausbeute: 1,7 g (82%), Schmp.: >250 °C, [α]_{D}²⁵ = (-) 43,0° (C=1 in Methanol).

Analog zu Beispiel 9 wird dargestellt:
4'-Methoxy-5,9-dimethyl-6,7-benzomorphan (**19**)

Man verwendet 4,8 g (18 mmol) N-Formyl-4'-methoxy-5,9-dimethyl-6,7-benzomorphan (**18**), 50 mL n-Propanol und 50 mL konz. Salzsäure. Man erhitzte 8 h unter Rückfluß. Ausbeute: 2,9 g (57%). Eine Probe wird mit Oxalsäure in das entsprechende Oxalat überführt **(HB)**, das einen Schmelzpunkt von 229 °C aufweist.

### Beispiel 10: (-)-(1 R,9α)-4'-Methoxy-5,9-dimethyl-6,7-benzomorphan ((-)-19)

9,5 g (41 mmol) 4'-Methoxy-5,9-dimethyl-6,7-benzomorphan (**19**) werden in 80 mL Ethanol gelöst und mit 6,2 g (41 mmol) R-(+)-Weinsäure versetzt. Die ausgefallenen Kristalle werden abgesaugt und zweimal aus Methanol umkristallisiert. Ausbeute 2,6 g ( 17%), Schmp.: 236 °C, ee > 98% (anhand der freien Base NMR-spektoskopisch durch Zugabe von Shiftreagenz bestimmt). Das Tartrat wird in Wasser gelöst, mit Kaliumcarbonat die Base freigesetzt und extrahiert zweimal mit 50 mL Essigester. Die vereinigten organischen Extrakte werden über MgSO₄ getrocknet und das Lösungsmittel im Vakuum abgezogen. Man erhält 1,6 g der freien Base.

### Beispiel 11: (-)-(1R,9α)-4'-Hydroxy-5,9-dimethyl-6,7-benzomorphan-hydrobromid ((-)-4aBr)

1,5 g (6,5 mmol) (-)-4'-Methoxy-5,9-dimethyl-6,7-benzomorphan ((-)-**19**) werden mit 15 mL 48%iger Bromwasserstoffsäure 2 h unter Rückfluß erhitzt. Anschließend wird im Vakuum eingeengt und der Rückstand mit THF diggeriert. Man erhält 1,5 g (78%) des gewünschten Produkts als Hydrobromid (amorpher Niederschlag).

Analog zu Beispiel 11 wird dargestellt:
4'-Hydroxy-9-methyl-6,7-benzomorphan-hydrobromid (**5a**Br)

9,5 g (31 mmol) 4'-Methoxy-9-methyl-6,7-benzomorphan-oxalat (**24**OX) werden zunächst in wenig Wasser gelöst und mit 7 g Kaliumcarbonat in die freie Base überführt. Man extrahiert dreimal mit je 200 mL Essigester und zieht das Lösungsmittel von der vereinigten organischen Phase ab. Anschließend wird mit 30 mL 48%iger Bromwasserstoffsäure versetzt. Man erhält 6,1 g (70%) des gewünschten Hydrobromids; Schmp.: 227 °C

### Beispiel 12: (-)-(1 R,9α,2"S)-2-(2"-Benzyloxy)propyl-4'-hydroxy-5,9-dimethyl-6,7-benzomorphan-hydrochlorid ((-)-4bCl)

0,75 g (2,5 mmol) (-)-4'-Hydroxy-5,9-dimethyl-6,7-benzomorphan-hydrobromide ((-)-**4a**Br) werden in 15 mL Dichlormethan suspendiert und mit 3 mL N-Methylmorpholin versetzt. Nach 30 min. kühlt man auf -5 °C und tropft langsam eine Lösung von 1,1 g (5,5 mmol) (-)-S-2-Benzyloxypropionsäurechlorid in 10 mL Dichlormethan hinzu. Man läßt noch 30 min. bei -5 °C rühren, versetzt mit 20 mL 2 N Salzsäure und trennt die organische Phase ab. Die organische Phase wird über MgSO₄ getrocknet, das Lösungsmittel im Vakuum abgezogen und der Rückstand in 40 mL THF aufgenommen. Zu dieser Lösung werden 0,5 g (13 mmol) LiAlH₄ hinzugegeben wobei die Temperatur auf 35 °C anstieg. Man läßt 30 min nachreagieren, versetzt mit 0,4 mL Wasser und 0,2 mL 5 N Natronlauge und trennt von anorganischen Niederschlag ab. Der Niederschlag wird mit 100 mL THF gewaschen und die vereinigten organischen Phasen im Vakuum eingeengt. Der Rückstand wird in 100 mL Ether aufgenommen, über MgSO₄ getrocknet und mit etherischer Salzsäure das Hydrochlorid gefällt. Die Kristalle werden abgetrennt und mit Aceton gewaschen. Ausbeute: 0.6 g (55%), Schmp.: 227 °C, [α]_{D}²⁵ = (-) 13,3° (C=1 in Methanol).

Analog zu Beispiel 12 werden dargestellt:
(-)-(1 R,9α,2"R)-2-(2"-Benzyloxy)propyl-4'-hydroxy-5,9-dimethyl-6,7-benzomorphan-hydrochlorid ((-)-**4c**Cl)

Es werden 0,75 g (2,5 mmol) (-)-4'-Hydroxy-5,9-dimethyl-6,7-benzomorphan-hydrobromid ((-)-**4a**Br) und 1,1 g (5,5 mmol) (+)-R-2-Benzyloxypropionsäurechlorid eingesetzt.
Ausbeute: 0,7 g (65%), Schmp.: 217 °C, [α]_{D}²⁵ = (-) 76,1° (C=1 in Methanol).
(-)-(1R,9α)-4'-Hydroxy-5,9-dimethyl-2-[2-(2-phenoxy)ethoxy]ethyl-6,7-benzomorphan-hydrochlorid ((-)-**4d**Cl)

Es werden 0,75 g (2,5 mmol) (-)-4'-Hydroxy-5,9-dimethyl-6,7-benzomorphan-hydrobromid ((-)-**4a**Br) und 1,1 g (5,5 mmol) Phenoxyethoxyacetylchlorid eingesetzt. Ausbeute: 0,2 g (20%) amorphes Pulver.
(-)-(1R,2"S)-2-(2"-Benzyloxy)propyl-4'-hydroxy-5,9,9-trimethyl-6,7-benzomorphan-hydrochlorid ((-)-**2b**Cl)

Es werden 1,6 g (6,9 mmol) (-)-4'-Hydroxy-5,9,9-trimethyl-6,7-benzomorphan ((-)-**2a**) und 2,3 g (11,6 mmol) (-)-S-2-Benzyloxypropionsäurechlorid eingesetzt. Ausbeute: 2,1 g (73%), Schmp.: 254 °C, [α]_{D}²⁵ = (-) 20,7° (C=1 in Methanol).
(+)-(1 S,2"R)-2-(2"-Benzyloxy)propyl-4'-hydroxy-5,9,9-trimethyl-6,7-benzomorphan-hydrochlorid ((+)-**2b**Cl)

Es werden 1,5 g (6,5 mmol) (+)-4'-Hydroxy-5,9,9-trimethyl-6,7-benzomorphan ((+)-**2a**) und 1,5 g (7,6 mmol) (+)-R-2-Benzyloxypropionsäurechlorid eingesetzt. Ausbeute: 1,4 g (52%), Schmp.: 256 °C, [α]_{D}²⁵ = (+) 20,3° (C=1 in Methanol).
(-)-(1R,2"R)-2-(2"-Benzyloxy)propyl-4'-hydroxy-5,9,9-trimethyl-6,7-benzomorphan-hydrochlorid ((-)-**2c**Cl)

Es werden 1,6 g (6,9 mmol) (-)-4'-Hydroxy-5,9,9-trimethyl-6,7-benzomorphan ((-)-**2a**) und 1,5 g (7,6 mmol) (+)-R-2-Benzyloxypropionsäurechlorid eingesetzt. Ausbeute: 1,7 g (59%), Schmp.: 245 °C, [α]_{D}²⁵ = (-) 96,5° (C=1 in Methanol).
(+)-(1S,2"S)-2-(2"-Benzyloxy)propyl-4'-hydroxy-5,9,9-trimethyl-6,7-benzomorphan-hydrochlorid
((+)-**2c**Cl)

Es werden 1,6 g (6,9 mmol) (+)-4'-Hydroxy-5,9,9-trimethyl-6,7-benzomorphan ((+)-**2a**) und 2,3 g (11,6 mmol) (-)-S-2-Benzyloxypropionsäurechlorid eingesetzt. Ausbeute: 2,0 g (70%), Schmp.: 245 °C, [α]_{D}²⁵ = (+) 97,8° (C=1 in Methanol).
(-)-(1R,2"S)-2-(2"-(2'"-Fluorobenzyl)oxy)propyl-4'-hydroxy-5,9,9-trimethyl-6,7-benzomorphan-hydrochlorid ((-)-**2d**Cl)

Es werden 0,8 g (3,4 mmol) (-)-4'-Hydroxy-5,9,9-trimethyl-6,7-benzomorphan ((-)-**2a**) und 1,4 g (6,5 mmol) (-)-S-2-(2'-Fluorobenzyl)oxypropionsäurechlorid eingesetzt. Ausbeute: 0,9 g (61 %), Schmp.: 212 °C, [α]_{D}²⁵ = (-) 24,7° (C=1 in Methanol).
(-)-( 1R,2"R)-2-(2"-(2'"-Fluorobenzyl)oxy)propyl-4'-hydroxy-5,9,9-trimethyl-6,7-benzomorphan-hydrochlorid ((-)-**2e**Cl)

Es werden 0,5 g (2,3 mmol) (-)-4'-Hydroxy-5,9,9-trimethyl-6,7-benzomorphan ((-)-**2a**) und 0,6 g (3,0 mmol) (+)-R-2-(2'-Fluorobenzyl)oxypropionsäurechlorid eingesetzt. Ausbeute: 0,7 g (70%), Schmp.: 145 °C, [α]_{D}²⁵ = (-) 88,4° (C=1 in Methanol).
(-)-(1R,2"S)-2-(2"-(4"'-Fluorobenzyl)oxy)propyl-4'-hydroxy-5,9,9-trimethyl-6,7-benzomorphan-hydrochlorid ((-)-**2f**Cl)

Es werden 0,8 g (3,4 mmol) (-)-4'-Hydroxy-5,9,9-trimethyl-6,7-benzomorphan ((-)-**2a**) und 1,4 g (6,5 mmol) (-)-S-2-(4'-Fluorobenzyl)oxypropionsäurechlorid eingesetzt. Ausbeute: 1,0 g (68%), Schmp.: 250 °C, [α]_{D}²⁵ = (-) 21,9° (C=1 in Methanol).
(-)-(1R,2"R)-2-(2"-(4'"-Fluorobenzyl)oxy)propyl-4'-hydroxy-5,9,9-trimethyl-6,7-benzomorphan-hydrochlorid ((-)-**2q**Cl)

Es werden 0,5 g (2,3 mmol) (-)-4'-Hydroxy-5,9,9-trimethyl-6,7-benzomorphan ((-)-**2a**) und 0,6 g (3,0 mmol) (+)-R-2-(4'-Fluorobenzyl)oxypropionsäurechlorid eingesetzt. Ausbeute: 0,6 g (58%), Schmp.: 128 °C, [α]_{D}²⁵ = (-) 95,4° (C=1 in Methanol).
(-)-(1R,2"S)-2-(2"-(2'",6'"-Difluorobenzyl)oxy)propyl-4'-hydroxy-5,9,9-trimethyl-6,7-benzomorphan-hydrochlorid ((-)-**2h**Cl)

Es werden 1,5 g (6,5 mmol) (-)-4'-Hydroxy-5,9,9-trimethyl-6,7-benzomorphan ((-)-**2a**) und 2,4 g (10,2 mmol) (-)-S-2-(2',6'-Difluorobenzyl)oxypropionsäurechlorid eingesetzt. Ausbeute: 2,0 g (68%), Schmp.: 245 °C, [α]_{D}²⁵ = (-) 272,3° (C=1 in Methanol).
(-)-(1R,2"S)-2-(2"-(2'",6'"-Dichlorobenzyl)oxy)propyl-4'-hydroxy-5,9,9-trimethyl-6,7-benzomorphan-hydrochlorid ((-)-**2i**Cl)

Es werden 2,3 g (10 mmol) (-)-4'-Hydroxy-5,9,9-trimethyl-6,7-benzomorphan ((-)-**2a**) und 3,2 g (12 mmol) (-)-S-2-(2',6'-Dichlorbenzyl)oxypropionsäurechlorid eingesetzt. Ausbeute: 2,8 g (58%), Schmp.: 260 °C, [α]_{D}²⁵ = (-) 14,1° (C=1 in Methanol).
(-)-(1 R,2"S)-2-(2"-(2'"-Methyl-benzyl)oxy)propyl-4'-hydroxy-5,9,9-trimethyl-6,7-benzomorphan-hydrochlorid ((-)-**2j**Cl)

Es werden 0,8 g (3,4 mmol) (-)-4'-Hydroxy-5,9,9-trimethyl-6,7-benzomorphan ((-)-**2a**) und 1,4 g (6,5 mmol) (-)-S-2-(2'-Methyl-benzyl)oxypropionsäurechlorid eingesetzt. Ausbeute: 0,8 g (55%), Schmp.: 249 °C, [α]_{D}²⁵ = (-) 10,9° (C=1 in Methanol).
(-)-(1R,2"S)-2-(2"-Cyclohexylmethoxy)propyl-4'-hydroxy-5,9,9-trimethyl-6,7-benzomorphan-hydrochlorid ((-)-**2k**Cl)

Es werden 1,9 g (8,2 mmol) (-)-4'-Hydroxy-5,9,9-trimethyl-6,7-benzomorphan ((-)-**2a**) und 2,0 g (10 mmol) (-)-S-2-(2'-Cyclohexylmethoxy)propionsäurechlorid eingesetzt. Ausbeute: 1,8 g (52%), Schmp.: 249 °C, [α]_{D}²⁵ = (-) 24,6° (C=1 in Methanol).
(-)-(1R,2"R)-2-(2"-Cyclohexylmethoxy)propyl-4'-hydroxy-5,9,9-trimethyl-6,7-benzomorphan-hydrochlorid ((-)-**2l**Cl)

Es werden 1,9 g (8,2 mmol) (-)-4'-Hydroxy-5,9,9-trimethyl-6,7-benzomorphan ((-)-**2a**) und 2,0 g (10 mmol) (+)-R-2-(2'-Cyclohexylmethoxy)propionsäurechlorid eingesetzt. Ausbeute: 1,7 g (49%), Schmp.: 140 °C, [α]_{D}²⁵ = (-) 92,2° (C=1 in Methanol).
(-)-(1R)-4'-Hydroxy-2-(5"-phenoxy)pentyl-5,9,9-trimethyl-6,7-benzomorphan-hydrochlorid ((-)-**2m**Cl)

Es werden 3,0 g (13 mmol) (-)-4'-Hydroxy-5,9,9-trimethyl-6,7-benzomorphan ((-)-**2a**) und 3,2 g (15 mmol) 5-Phenoxypentansäurechlorid eingesetzt. Ausbeute: 2,4 g (44%), Schmp.: 149 °C, [α]_{D}²⁵ = (-) 74,6° (C=1 in Methanol).
(-)-(1R)-4'-Hydroxy-2-(2"-(2'"-phenyl)ethoxy)ethyl-5,9,9-trimethyl-6,7-benzomorphan-hydrochlorid ((-)-**2n**Cl)

Es werden 2,0 g (8,7 mmol) (-)-4'-Hydroxy-5,9,9-trimethyl-6,7-benzomorphan ((-)-**2a**) und 2,2 g (11 mmol) 2-Phenylethoxyessigsäurechlorid eingesetzt. Ausbeute: 1,7 g (48%), Schmp.: 204 °C, [α]_{D}²⁵ = (-) 72,4° (C=1 in Methanol).
(-)-(1R)-4'-Hydroxy-2-(4"-phenoxy)butyl-5,9,9-trimethyl-6,7-benzomorphan-hydrochlorid ((-)-**2o**Cl)

Es werden 1,0 g (4,3 mmol) (-)-4'-Hydroxy-5,9,9-trimethyl-6,7-benzomorphan ((-)-**2a**) und 1,2 g (6 mmol) 4-Phenoxybuttersäurechlorid eingesetzt. Ausbeute: 0,7 g (39%), Schmp.: 250 °C, [α]_{D}²⁵ = (-) 82,8° (C=1 in Methanol).
(-)-(1R)-2-(2"-Benzyloxy)ethyl-4'-hydroxy-5,9,9-trimethyl-6,7-benzomorphan-hydrochlorid ((-)-**2p**Cl)

Es werden 2,3 g (10 mmol) (-)-4'-Hydroxy-5,9,9-trimethyl-6,7-benzomorphan ((-)-**2a**) und 4,3 g (22 mmol) 2-Benzyloxyacetylchlorid eingesetzt. Ausbeute: 2,5 g (62%), Schmp.: 253 °C, [α]_{D}²⁵ = (-) 78,1° (C=1 in Methanol).
(-)-(1R)-2-(2"-(2'",6'"-Difluorbenzyloxy)ethyl-4'-hydroxy-5,9,9-trimethyl-6,7-benzomorphan-hydrochlorid ((-)-**2q**Cl)

Es werden 1,2 g (5 mmol) (-)-4'-Hydroxy-5,9,9-trimethyl-6,7-benzomorphan ((-)-**2a**) und 1,1 g (5 mmol) 2-(2',6'-Difluorbenzyloxyacetylchlorid eingesetzt. Ausbeute: 1,5 g (68%), Schmp.: 246 °C, [α]_{D}²⁵ = (-) 71,0° (C=1 in Methanol).
(-)-(1R)-2-(3"-(2"',6"'-Difluorphenyl)propyl)-4'-hydroxy-5,9,9-trimethyl-6,7-benzomorphan-hydrochlorid ((-)-**2r**Cl)

Es werden 1,9 g (8,2 mmol) (-)-4'-Hydroxy-5,9,9-trimethyl-6,7-benzomorphan ((-)-**2a**) und 1,7 g (8,3 mmol) 3-(2',6'-Difluorphenylpropionsäurechlorid eingesetzt. Ausbeute: 1,6 g (46%), Schmp.: >250 °C, [α]_{D}²⁵ = (-) 68,6° (C=1 in Methanol).

### Beispiel 13: (+)-(1R,2"S)-2-(2"-Benzyloxy)propyl-4'-hydroxy-5-methyl-6,7-benzomorphan-hydrochlorid ((+)-5bCl) und (+)-(1S,2"S)-2-(2"-Benzyloxy)propyl-4'-hydroxy-5-methyl-6,7-benzomorphan-hydrochlorid ((+)-5cCl)

5,1 g (17 mmol) 4'-Hydroxy-9-methyl-6,7-benzomorphan-hydrobromid (**5a**Br) und 1,7 g (17 mmol) N-Methylmorpholin werden in 20 mL DMF gelöst und auf -5 °C gekühlt. Hierzu wird eine bei -5 °C hergestellte Lösung von 3,5 g (19 mmol) (-)-S-2-Benzyloxypropionsäure, 1,8 g (19 mmol) Chlorameisensäuremethylester und 2,0 g (19 mmol) N-Methylmorpholin in 20 mL Dichlormethan langsam zugetropft. Man läßt 1 h bei Raumtemperatur nachreagieren, zieht das Lösungsmittel im Vakuum weitgehend ab, nimmt in 60 mL Dichlormethan auf, extrahiert zweimal mit je 20 mL 2 N Salzsäure und einmal mit 20 mL Wasser. Die organische Phase wird über MgSO₄ getrocknet und das Lösungsmittel im Vakuum abgezogen. Nach Zugabe von wenig Ether kristallisiert das Produkt aus (Schmp.: 110 °C), wird abgesaugt und in 80 mL THF aufgenommen. Zu dieser Lösung werden 0,8 g (21 mmol) LiAlH₄ hinzugegeben wobei die Temperatur auf 35 °C ansteigt. Man läßt 1 h nachreagieren, versetzt mit 25 mL Wasser und 25 mL 40%ige Natriumtartratlösung. Die organische Phase wird abgetrennt, die wäßrige Phase zweimal mit je 100 mL Ether extrahiert und die vereinigten organischen Phasen im Vakuum eingeengt. Der Rückstand wird in 100 mL Ether aufgenommen, über Magnesiumsulfat getrocknet und mit etherischer Salzsäure das Hydrochlorid gefällt. Die Kristalle werden abgetrennt und aus i-Propanol umkristallisiert. Man erhält 1.1 g (17%) (+)-**5b**Cl, Schmp.: 246 °C, [α]_{D}²⁵ = (+) 11,8° (c=1 in Methanol). Die Mutterlauge wurde eingeengt, die Base freigesetzt und chromatographiert (300 g Kieselgel; Essigester/Cyclohexan 1:3). Es wird erneut mit etherischer Salzsäure das Hydrochlorid gefällt. Man erhält 0.3 g (5%) (+)-**5c**Cl, Schmp.: 241 °C, [α]_{D}²⁵ = (+) 52,4° (c=1 in Methanol).

Analog zu Beispiel 13 werden dargestellt:
(+)-(1 R,2"S)-2-[2"-(2'",6'"-Difluorbenzyloxy)]propyl-4'-hydroxy-5-methyl-6,7-benzomorphan-hydrochlorid ((+)-**5d**Cl) und (+)-(1S,2"S)-2-(2"-(2'",6'"-Difluorbenzyloxy))propyl-4'-hydroxy-5-methyl-6,7-benzomorphan-hydrochlorid ((+)-**5e**Cl)

Es werden 4,0 g (14 mmol) 4'-Hydroxy-5-methyl-6,7-benzomorphan-hydrobromid (**5a**Br) und 3,0 g (14 mmol) (+)-R-2-(2',6'-Difluorbenzyloxy)propionsäure eingesetzt. Man erhält 0.3 g (5%) (+)-**5d**Cl, Schmp.: 122 °C, [α]_{D}²⁵ = (+) 20,9° (c=1 in Methanol) und 1,8 g (30%) einer Mischung aus (+)-**5d**Cl und (+)-**5e**Cl, Schmp.: 194 °C, [α]_{D}²⁵ = (+) 42,2° (c=1 in Methanol).

### Beispiel 14: (-)-(1R)-4'-Hydroxy-5,9,9-trimethyl-2-[2"-(2'"-phenoxy)ethoxy]ethyl-6,7-benzomorphan-hydrochlorid ((-)-2uCl):

1,5 g (6,5 mmol) (-)-4'-Hydroxy-5,9,9-trimethyl-6,7-benzomorphan ((-)-**2a**) und 1,5 g (7,5 mmol) 2-(2-Phenoxy)ethoxy-ethylchlorid werden in 20 mL DMF gelöst, eine katalytische Menge KI und 1 g Kaliumcarbonat hinzugefügt. Die Mischung wird 5 h bei 130 °C gerührt, das Lösungsmittel im Vakuum abgezogen. Der Rückstand wird in 100 mL Wasser aufgenommen, dreimal mit je 100 mL Essigester extrahiert und die vereinigten organischen Extrakte werden noch einmal mit 50 mL Wasser gewaschen, über MgSO₄ getrocknet und das Lösungsmittel im Vakuum abgezogen. Der Rückstand wird in 40 mL Ether gelöst und mit etherischer Salzsäure das Hydrochlorid gefällt. Ausbeute: 1,4 g (50%), Schmp.: 190 °C, [α]_{D}²⁵ = (-) 81,1° (c=1 in Methanol).

Analog zu Beispiel 14 werden dargestellt:
(+)-(1 S)-4'-Hydroxy-5,9,9-trimethyl-2-[2"-(2'"-phenoxy)ethoxy]ethyl-6,7-benzomorphan-hydrochlorid ((+)-**2v**Cl):

Es wurden 1,5 g (6,5 mmol) (+)-4'-Hydroxy-5,9,9-trimethyl-6,7-benzomorphan ((+)-**2a**) und 1,5 g (7,5 mmol) 2-(2-Phenoxy)ethoxy-ethylchlorid eingesetzt. Ausbeute: 1,8 g (64%), Schmp.: 190 °C, [α]_{D}²⁵ = (+) 81,0° (C=1 in Methanol).
(-)-(1R,2"S)-2-[2"-(2'"-Cyanobenzyl)oxy]propyl-4'-hydroxy-5,9,9-trimethyl-6,7-benzomorphan-hydrochlorid ((-)-**2w**Cl)

Es werden 0,8 g (3,4 mmol) (-)-4'-Hydroxy-5,9,9-trimethyl-6,7-benzomorphan ((-)-**2a**) und 1,0 g (3,7 mmol) S-Methansulfonsäure-2-(2'-cyanobenzyl)oxypropylester eingesetzt. Ausbeute: 0,2 g (13%), Schmp.: 234 °C.
(-)-(1R)-2-[2"-(2'"-Cyclohexyloxy)ethoxy]ethyl-4'-hydroxy-5,9,9-trimethyl-6,7-benzomorphan-hydrochlorid ((-)-**2x**Cl)

Es werden 1,0 g (4,3 mmol) (-)-4'-Hydroxy-5,9,9-trimethyl-6,7-benzomorphan ((-)-**2a**) und 1,1 g (5,4 mmol) 2-(2'-Cyclohexyloxy)ethoxy)ethylchlorid eingesetzt. Ausbeute: 0,7 g (37%), Schmp.: 204 °C, [α]_{D}²⁵ = (-) 71,1° (C=1 in Methanol).
(-)-(1R)-2-{2"-[2'"-(2,6-Difluorphenoxy)ethoxy]ethyl}-4'-hydroxy-5,9,9-trimethyl-6,7-benzomorphan-hydrochlorid ((-)-**2y**Cl):

Es wurden 2,3 g (10 mmol) (-)-4'-Hydroxy-5,9,9-trimethyl-6,7-benzomorphan ((-)-**2a**) und 2,8 g (12 mmol) 2-(2'-(2,6-Difuorphenoxy)ethoxy-ethylchlorid eingesetzt. Ausbeute: 2,3 g (49%), Schmp.: 183 °C, [α]_{D}²⁵ = (-) 73,3° (C=1 in Methanol).
(-)-(1R)-2-[2"-(2,6-Difluorphenoxy)ethyl]-4'-hydroxy-5,9,9-trimethyl-6,7-benzomorphan-hydrochlorid ((-)-**2z**Cl):

Es werden 1,2 g (5 mmol) (-)-4'-Hydroxy-5,9,9-trimethyl-6,7-benzomorphan ((-)-**2a**) und 1,4 g (9,8 mmol) 2-(2,6-Difuorphenoxy)ethylchlorid eingesetzt. Ausbeute: 0,3 g (14%), Schmp.: 241 °C.
(-)-(1R)-2-(2"-Cylohexyloxy)ethyl-4'-hydroxy-5,9,9-trimethyl-6,7-benzomorphan-hydrochlorid ((-)-**2aa**Cl):

Es werden 1,2 g (5 mmol) (-)-4'-Hydroxy-5,9,9-trimethyl-6,7-benzomorphan ((-)-**2a**) und 1,1 g (6,8 mmol) 2-Cylohexyloxy-ethylchlorid eingesetzt. Ausbeute: 0,8 g (41%), Schmp.: >250 °C, [α]_{D}²⁵ = (-) 71,1° (C=1 in Methanol).
(-)-(R)-2-[2"-(2'"-tert.-Butyloxy)ethoxy]ethyl-4'-hydroxy-5,9,9-trimethyl-6,7-benzomorphan-hydrochlorid ((-)-**2ac**Cl)

Es werden 1,5 g (6,4 mmol) (-)-4'-Hydroxy-5,9,9-trimethyl-6,7-benzomorphan ((-)-**2a**) und 1,4 g (7,7 mmol) 2-(2'-tert.-Butyloxy)ethoxy)ethylchlorid eingesetzt. Ausbeute: 0,8 g (30%), Schmp.: 209 °C, [α]_{D}²⁵ = (-) 72,4° (C=1 in Methanol).

### Beispiel 15: (-)-(1R)-5-Ethyl-4'-hydroxy-9,9-dimethyl-2-(2-(2-phenoxy)ethoxy)ethyl-6,7-benzomorphan-hydrochlorid (3bCl):

1,0 g (3,4 mmol) (-)-5-Ethyl-4'-methoxy-9,9-dimethyl-6,7-benzomorphan (**10**) werden mit 20 mL 48%iger Bromwasserstoffsäure 2 h unter Rückfluß erhitzt. Anschließend wird im Vakuum eingeengt und der Rückstand zweimal mit je 20 mL Ethanol gelöst und wieder eingedampft. Anschließend wird in 20 mL DMF aufgenommen und 800 mg (4,0 mmol) 2-(2-Phenoxy)ethoxy-ethylchlorid in 10 mL DMF, eine katalytische Menge KI und 1 g Kaliumcarbonat hinzugefügt. Die Mischung wird 4 h bei 80 °C gerührt, das Lösungsmittel im Vakuum abgezogen. Der Rückstand wird in 100 mL Wasser aufgenommen, dreimal mit je 100 mL Essigester extrahiert und die vereinigten organischen Extrakte werden noch einmal mit 50 mL Wasser gewaschen, über MgSO₄ getrocknet und das Lösungsmittel im Vakuum abgezogen. Der Rückstand wird in 40 mL Ether gelöst und mit etherischer Salzsäure das Hydrochlorid gefällt. Ausbeute: 1,0 g (66%), Schmp.: 90 °C (Zers.).

### Beispiel 16: (-)-(1R)-4'-Hydroxy-2-(2"-phenylethyl)-5,9,9-trimethyl-6,7-benzomorphan-hydrochlorid ((-)-2adCl)

1,0 g (4,3 mmol) (-)-4'-Hydroxy-5,9,9-trimethyl-6,7-benzomorphan ((-)-**2a**) und 1,0 g (8,3 mmol) Phenylacetaldehyd werden in 20 mL Methanol gelöst, mit Molekularsieb versetzt und 3 h bei RT gerührt. Anschließend wird vom Molekularsieb abfiltriert und das Filtrat mit 0,6 g ( 9,5 mmol) Natriumcyanoborhydrid und 1,2 mL Eisessig versetzt. Man läßt ca. 12 Stunden stehen, versetzt mit 20 mL 4 N Salzsäure und engt im Vakuum ein. Der Rückstand wird mit wenig Aceton versetzt und die Kristalle abgesaugt. Ausbeute: 0,9 g (56%), Schmp.: 250 °C, [α]_{D}²⁵ = (-) 80,04° (C=1 in Methanol).

Analog zu Beispiel 16 wird dargestellt:
(-)-(1R)-4'-Hydroxy-2-(2"-phenylpropyl)-5,9,9-trimethyl-6,7-benzomorphan-hydrochlorid ((-)-**2ae**Cl)

Es wurden 1,0 g (4,3 mmol) (-)-4'-Hydroxy-5,9,9-trimethyl-6,7-benzomorphan ((-)-**2a**), 1,2 g (8,9 mmol) 3-Phenylpropionaldehyd und 0,6 g ( 9,5 mmol) Natriumcyanoborhydrid eingesetzt. Ausbeute: 0,8 g (48%), Schmp.: >250 °C, [α]_{D}²⁵ = (-) 75,1° (C=1 in Methanol).

### Beispiel 17: (-)-(1R)-4'-Hydroxy-2-[2"-(2'"-phenylamino)ethoxy]ethyl-5,9,9-trimethyl-6,7-benzomorphan-dihydrochlorid ((-)-2aeCl2)

1,3 g (5,6 mmol) (-)-4'-Hydroxy-5,9,9-trimethyl-6,7-benzomorphan ((-)-**2a**) und 1,7 g (5,6 mmol) 2-(N-Phenyl-2"-*tert*.-butoxycarbonylamino)ethoxyethylchlorid werden in 50 mL DMF gelöst, eine katalytische Menge KI und 1 g Kaliumcarbonat hinzugefügt. Die Mischung wird 7 h bei 110 °C gerührt, das Lösungsmittel im Vakuum abgezogen. Der Rückstand wird in 100 mL Wasser aufgenommen, dreimal mit je 100 mL Essigester extrahiert und die vereinigten organischen Extrakte werden noch einmal mit 50 mL Wasser gewaschen, über MgSO₄ getrocknet und das Lösungsmittel im Vakuum abgezogen. Der Rückstand wird über eine Flashsäule gereinigt (120 mL Kieselgel; Essigester/ Cyclohexan 1 :1 ) und mit 50 mL konz. Salzsäure 30 min. bei RT gerührt. Anschließend wird mit 150 mL Eiswasser verdünnt, einmal mit 50 mL Essigester ausgeschüttelt (organische Phase verworfen), und mit konz. Ammoniak alkalisch gestellt. Es wird dreimal mit je 100 mL Essigester extrahiert, die vereinigten organischen Extrakte über MgSO₄ getrocknet und das Lösungsmittel im Vakuum Abgezogen. Der Rückstand wird in 10 mL Ethanol gelöst und mit etherischer Salzsäure das Hydrochlorid gefällt. Ausbeute: 0,7 g (27%), Schmp.: 112 °C, [α]_{D}²⁵ = (-) 75,8° (C=1 in Methanol).

### Beispiel 19: (-)-(1R,2"S)-2-(2"-Benzyloxy)propyl-3'-chlor-4'-hydroxy-5,9,9-trimethyl-6,7-benzomorphan-hydrochlorid ((-)-2agCl) und (-)-(1R,2"S)-2-(2"-Benzyloxy)propyl-1'-chlor-4'-hydroxy-5,9,9-trimethyl-6,7-benzomorphan-hydrochlorid ((-)-2ahCl)

3 g (7,3 mmol) (-)-(1 R,2"S)-2-(2"-Benzyloxy)propyl-4'-hydroxy-5,9,9-trimethyl-6,7-benzomorphan-hydrochlorid ((-)-**2b**Cl) und 1,0 g (7,3 mmol) N-Chlorsuccinimid werden in 70 mL Eisessig suspendiert und 24 h bei Raumtemperatur gerührt wobei die Suspension in Lösung geht. Anschließend wird der Ansatz im Vakuum eingeengt, der Rückstand mit 100 mL eiskalter 2 N Natronlauge versetzt und dreimal mit je 100 mL Essigester ausgeschüttelt. Die vereinigten organischen Phasen werden über MgSO₄ getrocknet und das Lösungsmittel im Vakuum abgezogen. Anschließend wird der Rückstand an Kieselgel chromatographiert (180 g Kieselgel; Cyclohexan/Essigester 5: 1). Die geeigneten Fraktionen werden eingeengt und der Rückstand in 15 mL Aceton gelöst und mit etherischer Salzsäure das Hydrochlorid gefällt.
Man erhält 0.4 g (12%) (-)-**2ag**Cl, Schmp.: 204 °C, [α]_{D}²⁵ = (-) 21,5° (C=1 in Methanol) und erhält 0.6 g (18%) (-)-**2ah**Cl, Schmp.: 258 °C, [α]_{D}²⁵ = (-) 4,6° (C=1 in Methanol).

Im folgenden sind einige Beispiele für pharmazeutische Zubereitungen mit dem Wirkstoff angegeben:

| Tabletten: | |
|---|---|
| Wirkstoff gemäß allgemeiner Formel I | 20 mg |
| Magnesiumstearat | 1 mg |
| Laktose | 190 mg |

| Lösung zur Injektion | |
|---|---|
| Wirkstoff gemäß allgemeiner Formel I | 0,3 mg |
| Natriumchlorid | 0,8 g |
| Benzalkoniumchlorid | 0,01 mg |
| Aqua ad injectionem ad 100 ml | |

Eine ähnliche Lösung zu der oben aufgeführten ist geeignet für die nasale Applikation in einem Spray, oder in Kombination mit einem Gerät, das ein Aerosol mit einer Partikelgröße vorzugsweise zwischen 2 und 6 µM, zur Anwendung über die Lunge.

### Lösung zur Infusion

Eine 5 gewichtsprozentige Xylit-Lösung, die beispielseise den Wirkstoff in einer Konzentration von 2 mg/ml enthält, wird mit einem Natriumacetat-Puffer auf einen pH-Wert von ca. 4 eingsetsellt.

Derartige Infusionslösungen können einen Gehalt an Wirkstoff gemäß der allgeimenen Formel 1 bezogen auf die Gesamtmasse der pharmazeutischen Zubereitung in einem Bereich von 0,001 bis 20 Gew.-%, vorzugsweise in einem Bereich von 0,001 bis10 Gew.-% und besonders bevorzugt in einem Bereich von 0,01 bis 5 Gew.-% aufweisen.

### Kapseln für die Inhalation

Der Wirkstoff gemäß der allgemeinen Formel I wird in mikronisierter Form (Partikelgröße im wesentlichen zwischen 2 und 6 µM), gegebenfalls unter Zusatz von mikronisierten Trägersubstanzen, etwa Laktose, in Hartgelatinekapseln gefüllt. Zur Inhalation dienen übliche Geräte für die Pulverinhalation. In jede Kapsel werden z.B. zwischen 0,2 und 20 mg Wirkstoff und 0 bis 40 mg Laktose eingefüllt.

| Inhalationsaerosol | |
|---|---|
| Wirkstoff gemäß allgemeiner Formel I | 1 Teil |
| Sojalecithin | 0,2 Teile |
| Treibgasmischung ad | 100 Teile |

## Patentansprüche

1. Verbindungen der allgemeinen Formel (I) 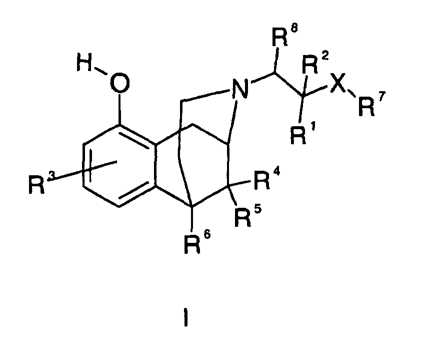 worin
X eine Einfachbindung, -O-, C₁-C₄-Alkylen, eine Alkylenbrücke mit 1 bis 8 Kohlenstoffatomen die verzeigt oder unverzweigt sein kann und an beliebiger Stelle in der Brücke ein oder zwei Sauerstoffatom(e) aufweisen kann, oder -O-CH₂-CH₂-NH-;
R¹ Wasserstoff, Methyl, Ethyl, Phenyl;
R2 Wasserstoff, Methyl;
R³ Wasserstoff, Fluor, Chlor, Brom, Hydroxy, Methyl, Methoxy;
R⁴ Wasserstoff, Methyl, Ethyl;
R⁵ Wasserstoff, Methyl, Ethyl;
R⁶ Wasserstoff, Methyl, Ethyl;
R⁷ tert.-Butyl, Cyclohexyl, Phenyl gegebenenfalls substittuiert durch R⁹ und R¹⁰, die gleich oder verschieden sein können, 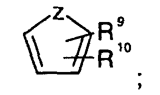
R⁸ Wasserstoff, C₁-C₄-Alkyl;
Z Sauerstoff, NH, Schwefel;
R⁹ Wasserstoff, Methyl, Fluor, Chlor, Brom, Methoxy;
R¹⁰ Wasserstoff, Methyl, Fluor, Chlor, Brom, Methoxy;
bedeuten können,
gegebenenfalls in Form der einzelnen optischen Isomeren, Mischungen der einzelnen Enantiomeren oder Racemate sowie in Form der freien Basen oder der enstsprechenden Säureadditionssalze mit pharmakologisch unbedenklichen Säuren.

2. Verbindungen der allgemeinen Formel I nach Anspruch 1, in der
X -O-C₁-C₃-Alkylen oder -O-CH₂-CH₂-O- ;
R¹ Wasserstoff, Methyl, Ethyl, Phenyl;
R² Wasserstoff, Methyl;
R³ Wasserstoff, Fluor, Chlor, Brom, Hydroxy, Methyl, Methoxy;
R⁴ Wasserstoff, Methyl, Ethyl;
R⁵ Wasserstoff, Methyl, Ethyl;
R⁶ Wasserstoff, Methyl, Ethyl;
R⁷ tert.-Butyl, Cyclohexyl, Phenyl gegebenenfalls substittuiert durch R⁹ und R¹⁰, die gleich oder verschieden sein können, 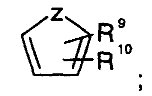
R⁸ Wasserstoff, C₁-C₄-Alkyl;
Z Sauerstoff, NH, Schwefel;
R⁹ Wasserstoff, Methyl, Fluor, Chlor, Brom, Methoxy;
R¹⁰ Wasserstoff, Methyl, Fluor, Chlor, Brom, Methoxy;
bedeuten können,
gegebenenfalls in Form der einzelnen optischen Isomeren, Mischungen der einzelnen Enantiomeren oder Racemate sowie in Form der freien Basen oder der enstsprechenden Säureadditionssalze mit pharmakologisch unbedenklichen Säuren.

3. Verbindung der Formel I nach Anspruch 1, **dadurch gekennzeichnet, daß** es sich um (-)-(1R,2"S)-2-(2"-Benzyloxy)propyl-4'-hydroxy-5,9,9-trimethyl-6,7-benzomorphan in Form der freien Basen oder der enstsprechenden Säureadditionssalze mit pharmakologisch unbedenklichen Säuren handelt.

4. Verbindung der Formel I nach Anspruch 1, **dadurch gekennzeichnet, daß** es sich um (-)-(1 R,2"S)-2-[2"-(2"',6"'-Difluorobenzyl)oxy]propyl-4'-hydroxy-5,9,9-trimethyl-6,7-benzomorphan in Form der freien Basen oder der enstsprechenden Säureadditionssalze mit pharmakologisch unbedenklichen Säuren handelt.

5. Verfahren zur Herstellung von Norbenzomorphanen der allgemeinen Formel 1 nach Anspruch 1, 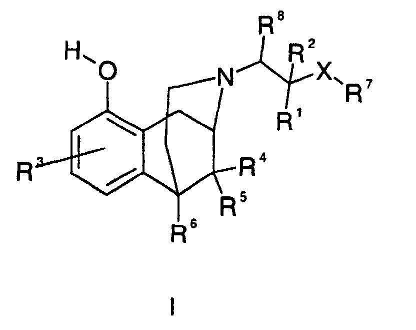 worin
X eine Einfachbindung, -O-, C₁-C₄-Alkylen, eine Alkylenbrücke mit 1 bis 8 Kohlenstoffatomen die verzeigt oder unverzweigt sein kann und an beliebiger Stelle in der Brücke ein oder zwei Sauerstoffatom(e) aufweisen kann, oder -O-CH₂-CH₂-NH-;
R¹ Wasserstoff, Methyl, Ethyl, Phenyl;
R² Wasserstoff, Methyl;
R³ Wasserstoff, Fluor, Chlor, Brom, Hydroxy, Methyl, Methoxy;
R⁴ Wasserstoff, Methyl, Ethyl;
R⁵ Wasserstoff, Methyl, Ethyl;
R⁶ Wasserstoff, Methyl, Ethyl;
R⁷ tert.-Butyl, Cyclohexyl, Phenyl gegebenenfalls substittuiert durch R⁹ und R¹⁰, die gleich oder verschieden sein können, 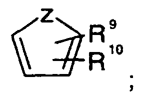
R⁸ Wasserstoff, C₁-C₄-Alkyl;
Z Sauerstoff, NH, Schwefel;
R⁹ Wasserstoff, Methyl, Fluor, Chlor, Brom, Methoxy;
R¹⁰ Wasserstoff, Methyl, Fluor, Chlor, Brom, Methoxy;
bedeuten können, **dadurch gekennzeichnet, daß** man
a) ein Benzylcyanid der allgemeinen Formel 32 in der R₃₀ eine C₁-C₄-Alkylgruppe bedeutet, mit einem Halogencarbonsäurearbonsäureester der allgemeinen Formel 33, in der R₄₀ C₁-C₈ -Alkyl oder Benzyl bedeutet, in Gegenwart eines Alkylhalogensilans - vorzugsweise einem Trialkylchlorsilan und besonders bevorzugt Trimethylchlorsilan - und Zinkpulver in einem inerten Lösungsmittel - bevorzugt in einem Ether oder in einem Halogenkohlenwasserstoff und besondersbevorzugt mit Dichlormethan und in Gegenwart eines hinsichtlich der Reduktion von Iminofunktionen selektiven Reduktionsmittels - vorzugsweise in Gegenwart eines Alkyliborhydridderivats und besonders bevorzugt in Gegenwart von Natriumcyanoborhydrid - den Bedingungen einer Reformatsky-Reaktion unterwirft und das resultierende Carbonsäuresäureeesterderivat der allgemeinen Formel 34 isoliert 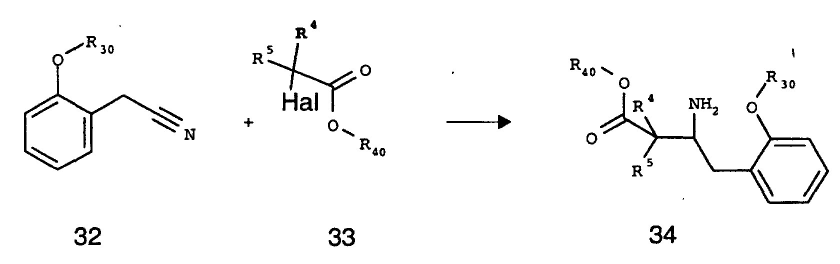 und
b) das Carbonsäureesterderivat der allgemeinen Formel 34 mit einem Acrylsäureester, in dem die Alkoholkomponente R₅₀ die Bedeutung einer C₁-C₈-Alkylgruppe oder einer Benzylgruppe hat, den Bedingungen einer Michael-Additionsreaktion in einem unter den gewählten Reaktionsbedingungen inerten Lösungsmittel - vorzugsweise in einem Alkanol und besonders bevorzugt in Ethanol - unterwirft und das resultierende Michael-Additionsprodukt der allgemeinen Formel 35 isoliert 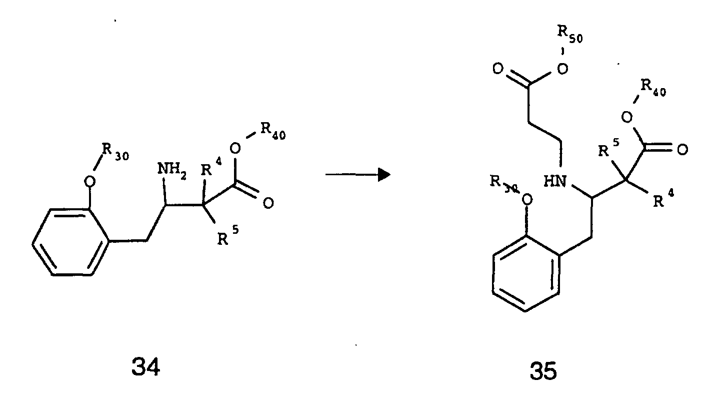 und
c) das so hergestellte Carbonsäurediesterderivat der allgemeinen Formel 35 in einem inerten Lösungsmittel - bevorzugt in einem aliphatischen oder aromatischen Kohlenwasserstoff und besonders bevorzugt in Toluol - in Gegenwart einer basisch reagierenden Verbindung - vorzugsweise in Gegenwart eines Alkalialkoholates eine verzweigten oder unverzweigten C₁-C₄-Alkanols und besonders bevorzugt in Gegenwart von Kalium-*tert*.-butanolat - den Bedingungen einer Dieckmann'schen Esterkondensation unterwirft und das resultierende Piperidonderivat der allgemeinen Formel 36 isoliert 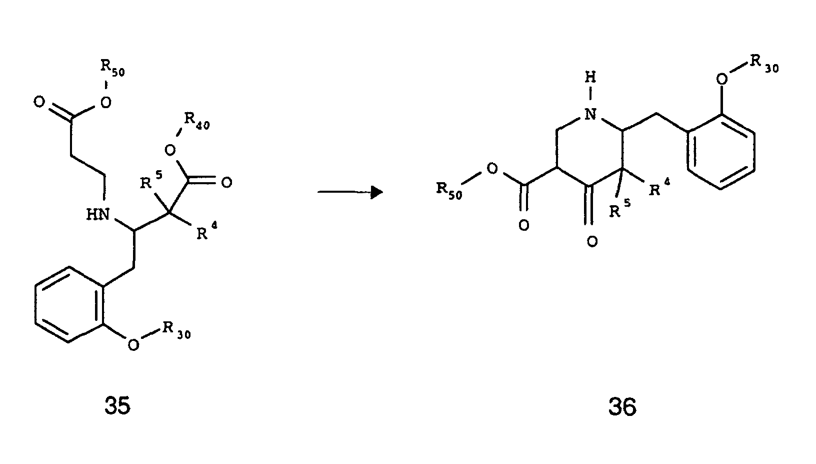 und
d) das Piperidonderivat 36 unter sauren oder alkalischen Bedingungen - bevorzugt in Gegenwart eines Alkalihydroxyds oder einer Mineralsäure und besonders bevorzugt in Gegenwart von Natriumhydroxyd - in einem polaren Lösungsmittel oder Lösungsmittelgemisch - bevorzugt in einer Mischung aus einem geradkettigen oder verzweigten C₁-C₄-Alkanol und Wasser und besonders bevorzugt in einem Ethanol/Wasser-Gemisch - unter Erhitzen zum entsprechenden Piperidonesterderivat der allgemeinen Formel 37 verseift und decarboxyliert, isoliert und gegebenenfalls mit einer Säure das entsprechende Säureadditionssalz herstellt und isoliert 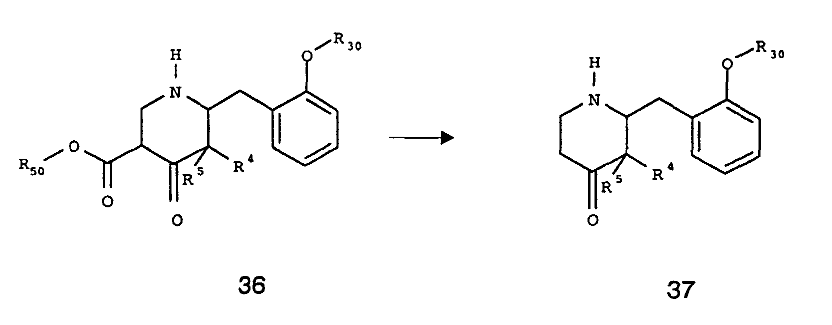 und
e) gewünschtenfalls das so erhaltene Stereoisomerengemisch gegebenenfalls nach Freisetzung der jeweiligen enantiomeren freien Basen in einem - hinsichtlich der Enantiomerentrennung - inerten Reaktionsmedium löst, mit einem geeigneten Stereoisomeren einer zur Salzbildung mit einem Stereoisomeren der Enantiomerenmischung geeigneten organischen Säure versetzt, das gewünschte Stereoisomere in Form seines Additionssalzes mit der optisch aktiven Säure isoliert. 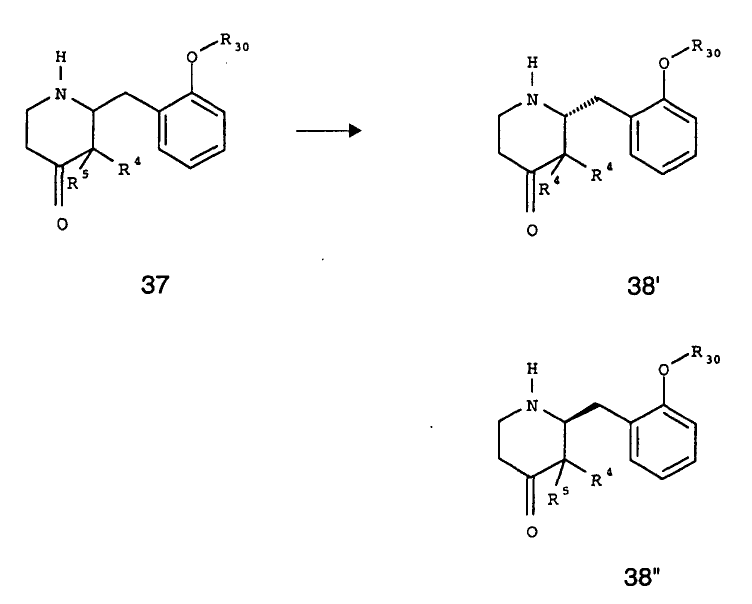 und
f) das so erhaltene reine Stereoisomere 38' bzw 38" oder das Isomerengemisch 38 nach dem Freisetzen aus dem enantiomerenreinen Säureadditionssalz in einem inerten Lösungsmittel mit einem eine CH₂= bzw.CH₃-CH= Gruppierung generierenden Wittig-Reagenz vorzugsweise mit einem Ethyltriphenylphosponiumhalogenid oder mit einem Methyltriphenylphosponiumhalogenid besonders bevorzug mit Methyltriphenylphosponiumbromid bzw. Ethyltriphenylphosponiumbromid in Gegenwart einer basischen reagierenden Verbindung - bevorzugt in Gegenwart eine Alkalialkoholats, besonders bevorzugt Kaliu-tert.-butanolat - in einem inerten Reaktionsmedium - bevorzugt in einem cyclischen Ether, besonders bevorzugt in Tetrahydrofuran - der Wittig-Reaktion unterwirft und das Reaktionsprodukt vom Typ 39 oder das entsprechende Strereoisomere gegebenenfalls in Form seines Säureadditionsalzes isoliert 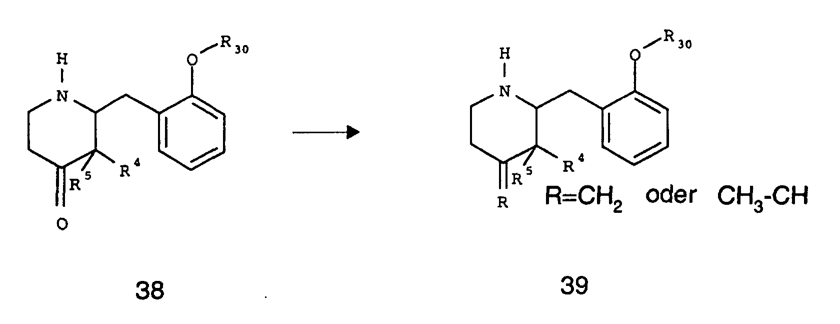 und
g) das aus der Wittig-Reaktion hervorgegangene Alken 39 gegebenenfalls zunächst aus seinem Säureadditionssalz freisetzt und die freie Base vom Typ 39 in einem organischen Lösungsmittel - bevorzugt in einem halogenierten Kohlenwasserstoff und besonders bevorzugt in Dichlormethan - löst und mit einem Formylierungsmittel - bevorzugt n-Butylformiat - einer Formylierungsreaktion am Piperidinstickstoff unterwirft und das Reaktionsprodukt vom Typ 40 oder das entsprechende Stereoisomere isoliert 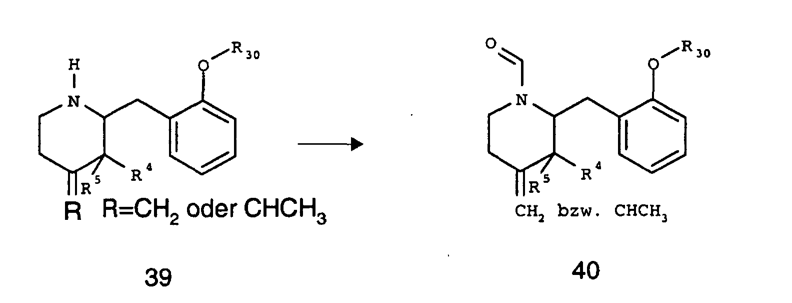 und
h) die so gewonnene Formylverbindung 40 - bzw. das entsprechende Stereoisomere - mit einer Mineralsäure oder mit einer Lewis-Säure - vorzugsweise Aluminium(III)chlorid - in einem inerten Lösungsmittel - vorzugsweise in einem halogenierten Kohlenwasserstoff und besonders bevorzugt in Dichlormethan - löst, umsetzt und das aus dieser Umsetzung resultierende Cyclisierungsprodukt vom Typ 41 isoliert 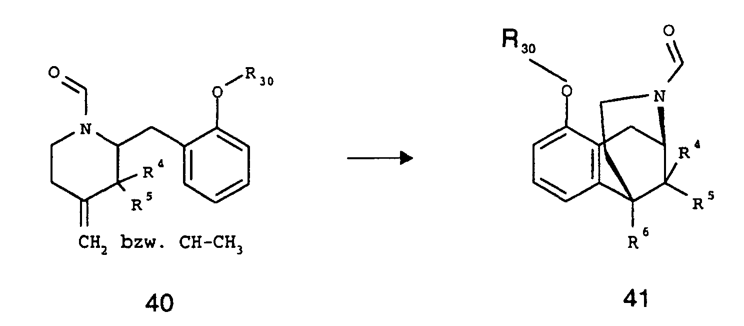 und
i) das aus der Cyclisierungsreaktion resultierende Benzomorphanderivat in einem polaren Lösungsmittel - vorzugsweise in einem C₁-C₄-Alkanol, besonders bevorzugt in n-Propanol - löst und mit einer sauer reagierenden Verbindung - bevorzugt mit der wässerigen Lösung einer Mineralsäure und besonders bevorzugt mit konzentrierter Salzsäure - zur Reaktion bringt und das aus dieser Umsetzung resultierende deformylierte Norbenzomorphan vom Typ 42 gegebenenfalls in Form seines Säureadditionssalzes isoliert 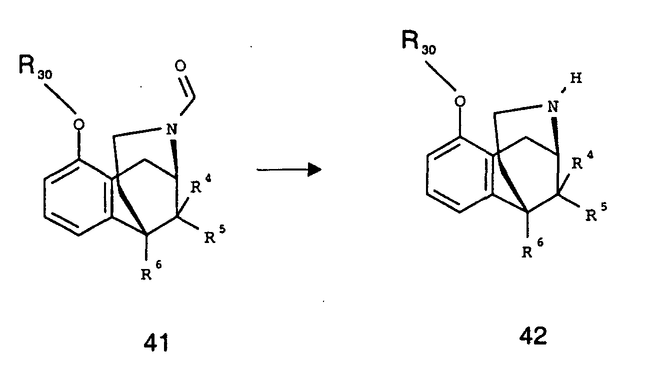 und
j) falls noch keine Trennung der Stereoisomeren erfolgte auf dieser Stufe die Stereoisomeren auf ans sich bekannte Weise trennt und nach Freisetzung der freien Benzomorphan-Base den Phenolether mit einer sauer reagierenden Verbindung - vorzugsweise mit einer Mineralsäure und besonders bevorzugt mit Bromwasserstoffsäure - spaltet und das Spaltprodukt vom Typ 43 isoliert 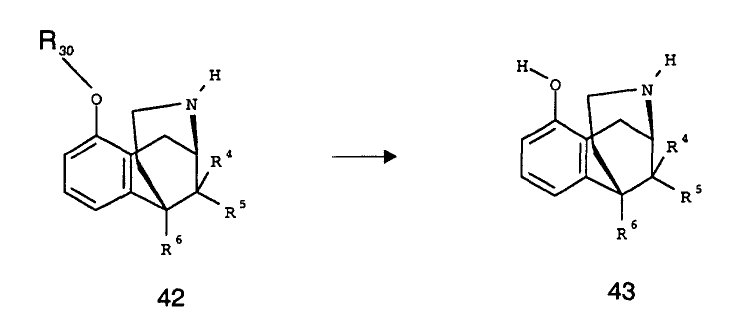
k) mit einer Verbindung vom Typ Z-CHR₈-R' umsetzt in der Z eine durch einen sekundären Aminstickstoff substituierbare Austrittsgruppe bedeutet - bevorzugt Halogen wie Chlor, Brom, lod oder ein organisches Sulfonat, bevorzugt Trifluormethansulfonat und R' die Bedeutung -CR¹R²XR⁷ hat, 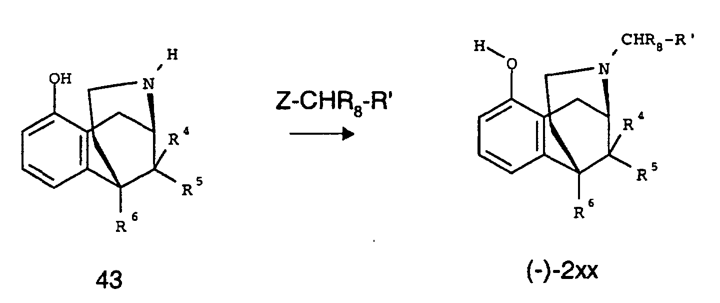 oder
mit einer Verbindung vom Typ YC(O)R' umsetzt, in der Ydurch einen sekundären Aminstickstoff substituierbare Austrittsgruppe bedeutet - bevorzugt Halogen wie Chlor, Brom, lod oder ein organisches Sulfonat, bevorzugt Trifluormethansulfonat und R' die Bedeutung -CR¹R²XR⁷ hat, und anschließend die Carbonylverbindung zur Verbindung (-)-2xx reduziert 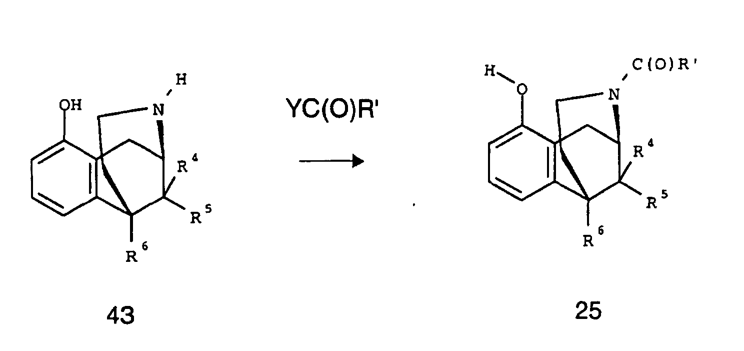 oder
mit einem Aldehyd der allgemeinen Formel HC(O)-R' umsetzt, in der R' die Bedeutung -CR¹R²XR⁷ hat, und die resultierende Schiff'sche Base 26 zur Verbindung (-)-2xx reduziert 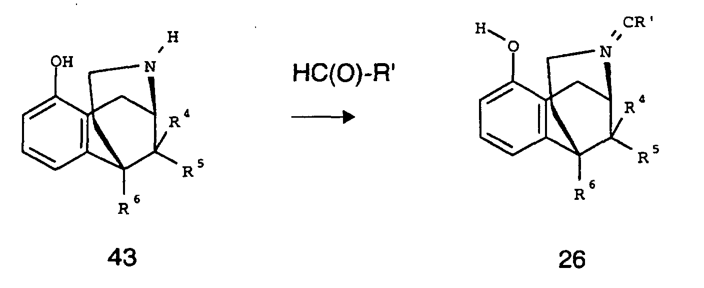 und
I) gegebenenfalls im Rahmen einer elektrophilen Substitution den Substitueneten R³ einführt

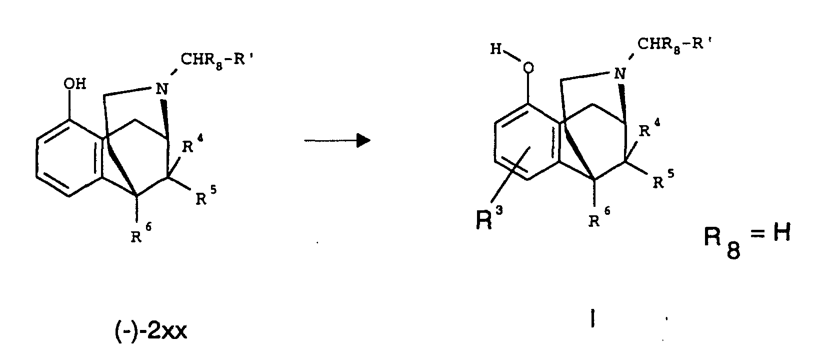

6. Pharmazeutische Zubereitung **gekennzeichnet durch** einen Gehalt an einer der Verbindungen nach einem der Ansprüche 1 bis 4 und deren Säureadditionssalze neben üblichen Hilfs- und Trägerstoffen.

7. Pharmazeutische Zubereitung zur nach Anspruch 6, **dadurch gekennzeichnet, daß** sie als Infusionslösung formuliert ist.

8. Pharmazeutische Zubereitung nach Anspruch 7, **dadurch gekennzeichnet, daß** der Wirkstoffgehalt bezogen auf die Gesamtmasse der pharmazeutischen Zubereitung in einem Bereich von 0,001 bis 20 Gew.-% liegt.

9. Pharmazeutische Zubereitung nach Anspruch 8, **dadurch gekennzeichnet, daß** der Wirkstoffgehalt bezogen auf die Gesamtmasse der pharmazeutischen Zubereitung in einem Bereich von 0,001 bis 10 Gew.-% liegt.

10. Pharmazeutische Zubereitung nach Anspruch 9, **dadurch gekennzeichnet, daß** der Wirkstoffgehalt bezogen auf die Gesamtmasse der pharmazeutischen Zubereitung in einem Bereich von 0,01 bis 5 Gew.-% liegt.

11. Verwendung von Verbindungen nach einem der Ansprüche 1 bis 4 als Arzneimittel.

12. Verwendung von Verbindungen nach einem der Ansprüch 1 bis 4 zur Herstellung eines Arzneimittels zur therapeutischen Behandlung von Gehirnischämien verschiedener Genese, neurodegenerativen Erkrankungen, Status epileptikus, Hypoglykämie, Hypoxie, Anoxie, Gehirntrauma, Gehirnoedem, Gehirn-Schlaganfall, perinatale Asphyxie, amylotrope laterale Sklerose, Huntington's Disease, Morbus Alzheimer, Morbus Parkinson, Bipolar Disorders, Zyklophrenie, Hypotonie, Herzinfakt, Herzrhythmusstörungen, Angina pectoris, Schmerzen zur Anaesthesie oder Lokalanaesthesie.

## Claims

1. Compounds of general formula (I) 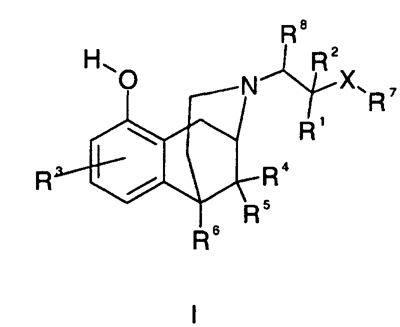 wherein
X denotes a single bond, -O-, C₁-C₄-alkylene, an alkylene bridge having 1 to 8 carbon atoms which may be branched or unbranched and may have one or two oxygen atom(s) anywhere in the bridge, or -O-CH₂-CH₂-NH;
R¹ denotes hydrogen, methyl, ethyl, phenyl;
R² denotes hydrogen, methyl;
R³ denotes hydrogen, fluorine, chlorine, bromine, hydroxy, methyl, methoxy;
R⁴ denotes hydrogen, methyl, ethyl;
R⁵ denotes hydrogen, methyl, ethyl;
R⁶ denotes hydrogen, methyl, ethyl;
R⁷ denotes tert.-butyl, cyclohexyl, phenyl optionally substituted by R⁹ and R¹⁰, which may be identical or different, 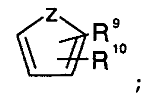
R⁸ denotes hydrogen, C₁-C₄-alkyl;
Z denotes oxygen, NH, sulphur;
R⁹ denotes hydrogen, methyl, fluorine, chlorine, bromine, methoxy;
R¹⁰ denotes hydrogen, methyl, fluorine, chlorine, bromine, methoxy;
optionally in the form of the individual optical isomers, mixtures of the individual enantiomers or racemates as well as in the form of the free bases or the corresponding acid addition salts with pharmaceutically acceptable acids.

2. Compounds of general formula I according to claim 1, wherein
X denotes O-C₁-C₃-alkylene or -O-CH₂-CH₂-O-;
R¹ denotes hydrogen, methyl, ethyl or phenyl;
R² denotes hydrogen or methyl;
R³ denotes hydrogen, fluorine, chlorine, bromine, hydroxy, methyl, methoxy;
R⁴ denotes hydrogen, methyl, ethyl;
R⁵ denotes hydrogen, methyl, ethyl;
R⁶ denotes hydrogen, methyl, ethyl;
R⁷ denotes tert.-butyl, cyclohexyl, phenyl optionally substituted by R⁹ and R¹⁰, which may be identical or different, 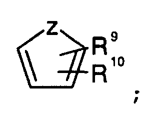
R⁸ denotes hydrogen, C₁-C₄-alkyl;
Z denotes oxygen, NH, sulphur;
R⁹ denotes hydrogen, methyl, fluorine, chlorine, bromine, methoxy;
R¹⁰ denotes hydrogen, methyl, fluorine, chlorine, bromine, methoxy;
optionally in the form of the individual optical isomers, mixtures of the individual enantiomers or racemates and also in the form of the free bases or the corresponding acid addition salts with pharmacologically acceptable acids.

3. Compound of formula I according to claim 1, **characterised in that** it is (-)-(1R,2"S)-2-(2"-benzyloxy)propyl-4'-hydroxy-5,9,9-trimethyl-6,7-benzomorphan in the form of the free bases or the corresponding acid addition salts with pharmacologically acceptable acids.

4. Compound of formula I according to claim 1, **characterised in that** it is (-)-(1R,2"S)-2-[2"-(2' ",6' "-difluorobenzyl)-oxy]propyl-4'-hydroxy-5,9,9-trimethyl-6,7-benzomorphan in the form of the free bases or the corresponding acid addition salts with pharmacologically acceptable acids.

5. Process for preparing norbenzomorphans of general formula 1 according to claim 1, 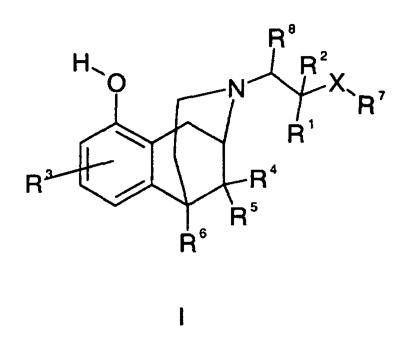 wherein
X denotes a single bond, -O-, C₁-C₄-alkylene, an alkylene bridge having 1 to 8 carbon atoms which may be branched or unbranched and may have one or two oxygen atom(s) anywhere in the bridge, or -O-CH₂-CH₂-NH;
R¹ denotes hydrogen, methyl, ethyl, phenyl;
R² denotes hydrogen, methyl;
R³ denotes hydrogen, fluorine, chlorine, bromine, hydroxy, methyl, methoxy;
R⁴ denotes hydrogen, methyl, ethyl;
R⁵ denotes hydrogen, methyl, ethyl;
R⁶ denotes hydrogen, methyl, ethyl;
R⁷ denotes tert.-butyl, cyclohexyl, phenyl optionally substituted by R⁹ and R¹⁰, which may be identical or different, 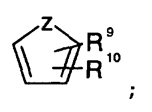
R⁸ denotes hydrogen, C₁-C₄-alkyl;
Z denotes oxygen, NH, sulphur;
R⁹ denotes hydrogen, methyl, fluorine, chlorine, bromine, methoxy;
R¹⁰ denotes hydrogen, methyl, fluorine, chlorine, bromine, methoxy;
**characterised in that**
a) a benzylcyanide of general formula 32 wherein R₃₀ denotes a C₁-C₄-alkyl group is subjected to the conditions of a Reformatsky reaction with a halocarboxylate of general formula 33, wherein R₄₀ denotes C₁-C₈-alkyl or benzyl, in the presence of an alkylhalosilane - preferably a trialkylchlorosilane and most preferably trimethylchlorosilane - and zinc powder in an inert solvent - preferably in an ether or in a halohydrocarbon and most preferably with dichloromethane and in the presence of a reducing agent which is selective with regard to the reduction of imino functions - preferably in the presence of an alkali metal borohydride derivative and most preferably in the presence of sodium cyanoborohydride - and the resulting carboxylic acid ester derivative of general formula 34 is isolated 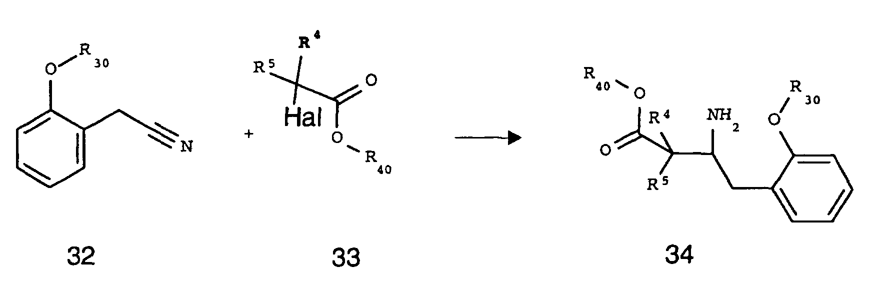 and
b) the carboxylic acid ester derivative of general formula 34 is subjected to the conditions of a Michael addition reaction with an acrylic acid ester, wherein the alcohol component R₅₀ denotes a C₁-C₈-alkyl group or a benzyl group, in a solvent which is inert under the reaction conditions selected - preferably in an alkanol and most preferably in ethanol - and the resulting Michael addition product of general formula 35 is isolated 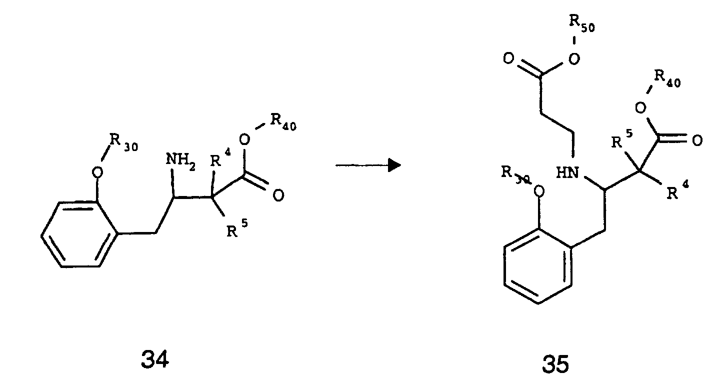 and
c) the carboxylic acid diester derivative of general formula 35 thus prepared is subjected to the conditions of Dieckmann's ester condensation in an inert solvent - preferably in an aliphatic or aromatic hydrocarbon and most preferably in toluene - in the presence of a basically reacting compound - preferably in the presence of an alkali metal alkoxide, a branched or unbranched C₁-C₄-alkanol and most preferably in the presence of potassium-*tert*.-butoxide - and the resulting piperidone derivative of general formula 36 is isolated 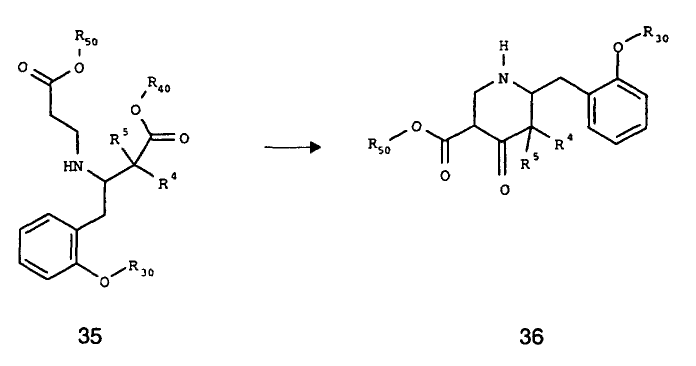 and
d) the piperidone derivative 36 is saponified and decarboxylated under acid or alkaline conditions - preferably in the presence of an alkali metal hydroxide or an inorganic acid and most preferably in the presence of sodium hydroxide - in a polar solvent or solvent mixture - preferably in a mixture of a straight-chain or branched C₁-C₄-alkanol and water and most preferably in an ethanol/water mixture - with heating, to obtain the corresponding piperidone ester derivative of general formula 37, which is isolated, and optionally the corresponding acid addition salt is prepared with an acid and isolated 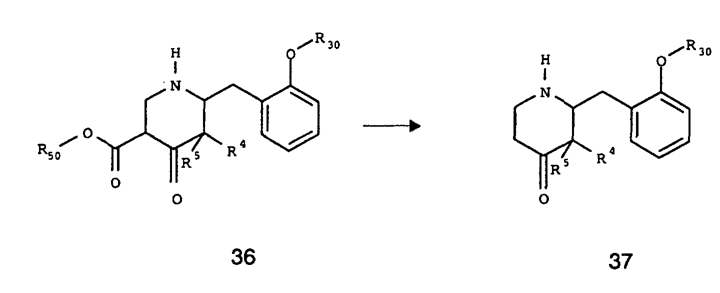 and
e) if desired, the mixture of stereoisomers thus obtained is dissolved in a reaction medium which is inert with regard to the enantiomer separation, optionally after the release of the enantiomeric free bases, combined with a suitable stereoisomer of an organic acid suitable for salt formation with a stereoisomer of the enantiomer mixture, and the desired stereoisomer is isolated in the form of its addition salt with the optically active acid 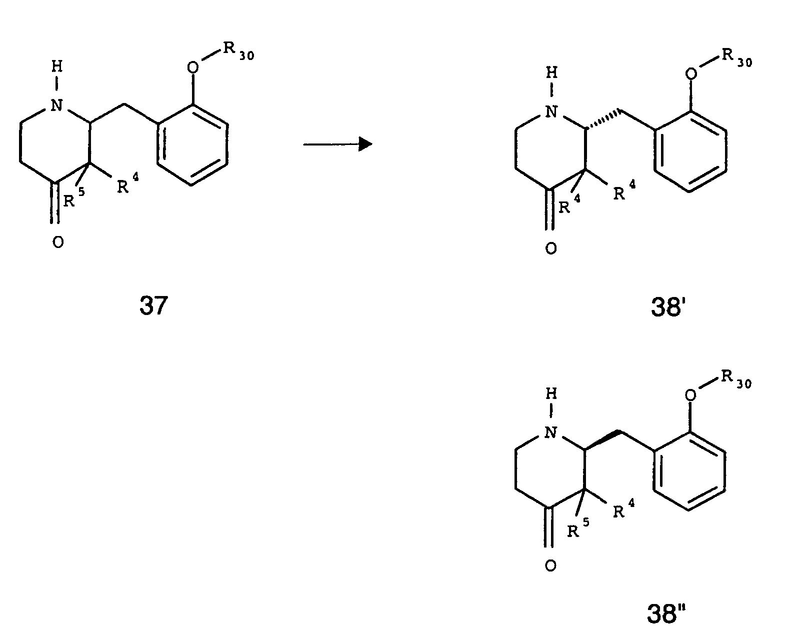 and
f) the pure stereoisomer 38' or 38" or the isomer mixture 38 thus obtained is subjected to a Wittig reaction after liberation from the enantiomerically pure acid addition salt in an inert solvent with a Wittig reagent generating a CH₂= or CH₃-CH= grouping, preferably with an ethyltriphenylphosphonium halide or with a methyltriphenylphosphonium halide, most preferably with methyltriphenylphosphonium bromide or ethyltriphenylphosphonium bromide in the presence of a basically reacting compound - preferably in the presence of an alkali metal alkoxide, most preferably potassium tert.-butoxide - in an inert reaction medium - preferably in a cyclic ether, most preferably in tetrahydrofuran - and the reaction product of type 39 or the corresponding stereoisomer is optionally isolated in the form of its acid addition salt 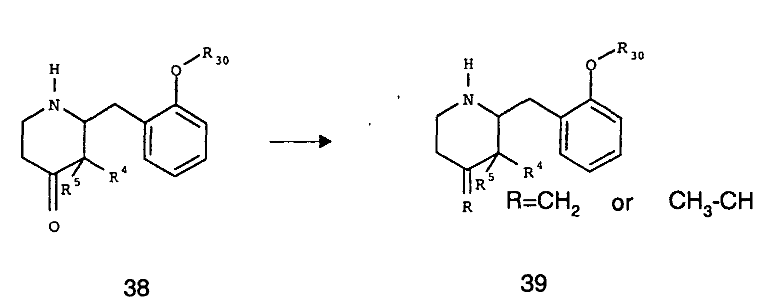 and
g) the alkene 39 obtained from the Wittig reaction is optionally first liberated from the acid addition salt thereof and the free base of type 39 is dissolved in an organic solvent - preferably in a halogenated hydrocarbon and most preferably in dichloromethane - and subjected to a formylation reaction with a formylating agent - preferably n-butylformate - at the piperidine nitrogen and the reaction product of type 40 or the corresponding stereoisomer is isolated 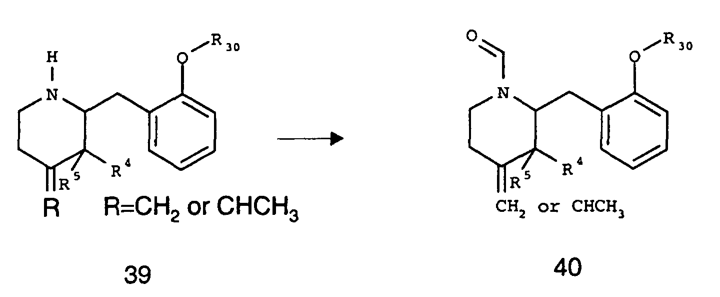 and
h) the formyl compound 40 thus obtained - or the corresponding stereoisomer - is reacted with an inorganic acid or with a Lewis acid - preferably aluminium(III)chloride - dissolved in an inert solvent - preferably in a halogenated hydrocarbon and most preferably in dichloromethane - and the cyclisation product of type 41 resulting from this reaction is isolated 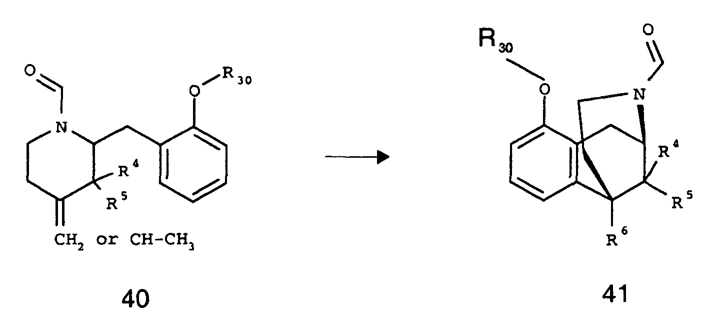 and
i) the benzomorphan derivative resulting from the cyclisation reaction is dissolved in a polar solvent - preferably in a C₁-C₄-alkanol, most preferably in n-propanol - and reacted with an acidically reacting compound - preferably with the aqueous solution of an inorganic acid and most preferably with concentrated hydrochloric acid - and the deformylated norbenzomorphan of type 42 resulting from this reaction is isolated, optionally in the form of its acid addition salt 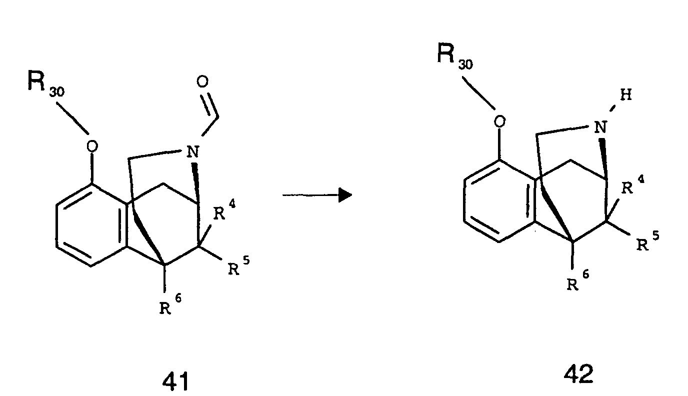 and
j) if the stereoisomers have not yet been separated, at this stage the stereoisomers are separated in a manner known per se and after the release of the free benzomorphan base the phenolether is cleaved with an acidically reacting compound - preferably with an inorganic acid and most preferably with hydrobromic acid - and the cleavage product of type 43 is isolated 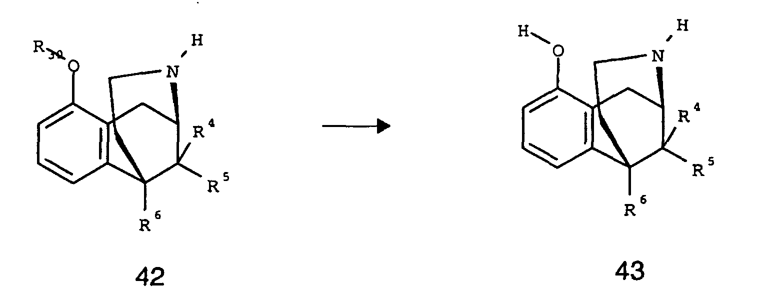
k) reacted with a compound of type Z-CHR₈-R' wherein Z denotes a secondary amino nitrogen substituted leaving group, preferably a halogen such as chlorine, bromine, iodine or an organic sulphonate, preferably trifluoromethanesulphonate, and R' denotes -CR¹R²XR⁷, 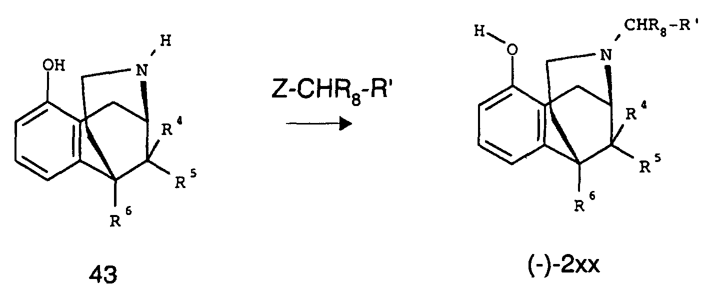 or
is reacted with a compound of type YC(O)R' wherein Y denotes a secondary amino nitrogen substituted leaving group, preferably a halogen such as chlorine, bromine, iodine or an organic sulphonate, preferably trifluoromethanesulphonate, and R' denotes -CR¹R²XR⁷, and subsequently the carbonyl compound is reduced to the compound (-)-2xx 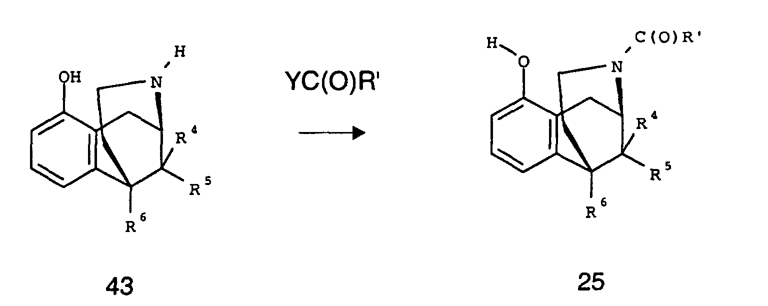 or
reacted with an aldehyde of general formula HC(O)-R', wherein R' denotes -CR¹R²XR⁷, and the resulting Schiff base 26 is reduced to the compound (-)-2xx 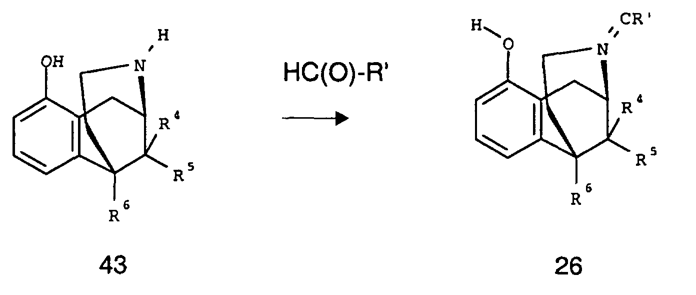 and
l) optionally within the framework of an electrophilic substitution the substituent R³ is introduced

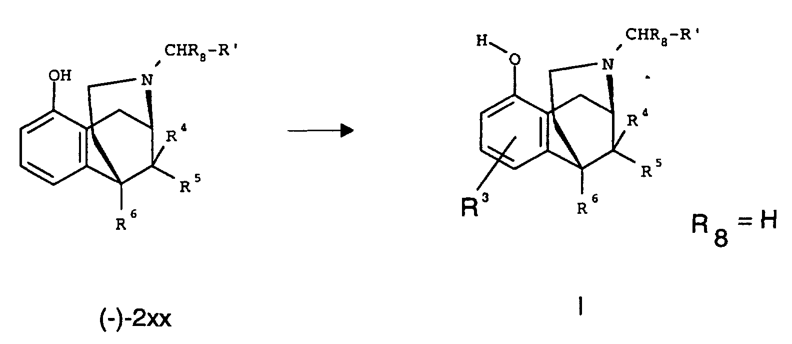

6. Pharmaceutical preparation **characterised in that** it contains one of the compounds according to one of claims 1 to 4 and the acid addition salts thereof together with conventional adjuvants and carriers.

7. Pharmaceutical preparation according to claim 6, **characterised in that** it is formulated as a solution for infusion.

8. Pharmaceutical preparation according to claim 7, **characterised in that** the content of active substance, based on the total mass of the pharmaceutical preparation, is in a range from 0.001 to 20 % by weight.

9. Pharmaceutical preparation according to claim 8, **characterised in that** the content of active substance, based on the total mass of the pharmaceutical preparation, is in a range from 0.001 to 10 % by weight.

10. Pharmaceutical preparation according to claim 9, **characterised in that** the content of active substance, based on the total mass of the pharmaceutical preparation, is in a range from 0.01 to 5 % by weight.

11. Use of compounds according to one of claims 1 to 4 as medicaments.

12. Use of compounds according to one of claims 1 to 4 for preparing a pharmaceutical composition for the therapeutic treatment of cerebral ischaemias of various origins, neurodegenerative disorders, epilepsy, hypoglycaemia, hypoxia, anoxia, brain trauma, cerebral oedema, cerebral stroke, perinatal asphyxia, amylotropic lateral sclerosis, Huntington's disease, Alzheimer's disease, Parkinson's disease, bipolar disorders, cyclophrenia, hypotonia, cardiac infarct, cardiac rhythm disorders, angina pectoris, or pain, for anaesthesia and local anaesthesia.

## Revendications

1. Composés de la formule générale (I) : 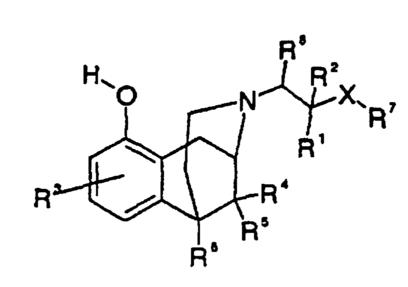 dans laquelle :
X peut représenter une liaison simple, -O-, un alkylène en C₁-C₄, un pont alkylène avec 1 à 8 atomes de carbone, qui peut être ramifié ou non ramifié, et peut comprendre sur un site quelconque du pont, un ou deux atomes d'oxygène, ou -O-CH₂-CH₂-NH- ;
R¹ peut représenter hydrogène, méthyle, éthyle, phényle ;
R² peut représenter hydrogène, méthyle ;
R³ peut représenter hydrogène, fluor, chlore, brome, hydroxyle, méthyle, méthoxy ;
R⁴ peut représenter hydrogène, méthyle, éthyle ;
R⁵ peut représenter hydrogène, méthyle, éthyle ;
R⁶ peut représenter hydrogène, méthyle, éthyle ;
R⁷ peut représenter t-butyle, cyclohexyle, phényle, la cas échéant, substitué par R⁹ et R¹⁰, qui peuvent être identiques ou différents, 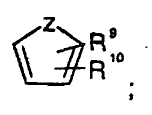
R⁸ peut représenter hydrogène, alkyle en C₁-C₄ ;
Z peut représenter oxygène, NH, soufre ;
R⁹ peut représenter hydrogène, méthyle, fluor, chlore, brome, méthoxy ;
R¹⁰ peut représenter hydrogène, méthyle, fluor, chlore, brome, méthoxy ;
le cas échéant sous la forme des isomères optiques individuels, des mélanges des énantiomères individuels ou des racémates, ainsi que sous la forme des bases libres ou des sels d'addition acide correspondants avec des acides pharmacologiquement inoffensifs.

2. Composés de la formule générale I selon la revendication 1, où
X peut représenter -O-alkylène en C₁-C₃ ou -O-CH₂-CH₂-O- ;
R¹ peut représenter hydrogène, méthyle, éthyle, phényle ;
R² peut représenter hydrogène, méthyle ;
R³ peut représenter hydrogène, fluor, chlore, brome, hydroxyle, méthyle, méthoxy ;
R⁴ peut représenter hydrogène, méthyle, éthyle ;
R⁵ peut représenter hydrogène, méthyle, éthyle ;
R⁶ peut représenter hydrogène, méthyle, éthyle ;
R⁷ peut représenter t-butyle, cyclohexyle, phényle, la cas échéant, substitué par R⁹ et R¹⁰, qui peuvent être identiques ou différents, 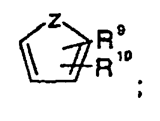 R⁸ peut représenter hydrogène, alkyle en C₁-C₄ ;
Z peut représenter oxygène, NH, soufre ;
R⁹ peut représenter hydrogène, méthyle, fluor, chlore, brome, méthoxy ;
R¹⁰ peut représenter hydrogène, méthyle, fluor, chlore, brome, méthoxy ;
le cas échéant sous la forme des isomères optiques individuels, des mélanges des énantiomères individuels ou des racémates, ainsi que sous la forme des bases libres ou des sels d'addition acide correspondants avec des acides pharmacologiquement inoffensifs.

3. Composés de la formule I selon la revendication 1, **caractérisé en ce qu'**il s'agit du (-)-(1R,2''S)-2-(2''-benzyloxy)propyl-4'-hydroxy-5,9,9-triméthyl-6,7-benzomorphane sous la forme de la base libre ou du sel d'addition acide correspondant avec des acides pharmacologiquement inoffensifs.

4. Composés de la formule I selon la revendication 1, **caractérisé en ce qu'**il s'agit du (-)-(1R,2''S)-2-[2''-(2''',6'''-difluorobenzyl)oxy]propyl-4'-hydroxy-5,9,9-triméthyl-6,7-benzomorphane sous la forme de la base libre ou du sel d'addition acide correspondant avec des acides pharmacologiquement sans inconvénients.

5. Procédé de préparation de norbenzomorphanes de la formule générale I selon la revendication 1 : 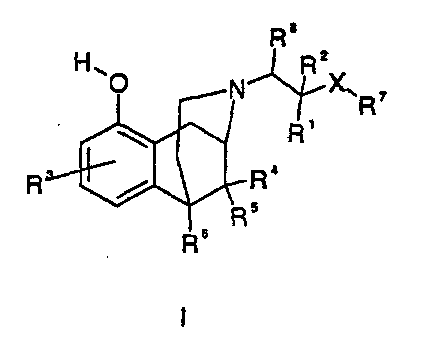 dans laquelle :
X peut représenter une liaison simple, -O- alkylène en C₁-C₄, un pont alkylène avec 1 à 8 atomes de carbone, qui peut être ramifié ou non ramifié, et peut comprendre sur un site quelconque du pont, un ou deux atomes d'oxygène, ou -O-CH₂-CH₂-NH- ;
R¹ peut représenter hydrogène, méthyle, éthyle, phényle ;
R² peut représenter hydrogène, méthyle ;
R³ peut représenter hydrogène, fluor, chlore, brome, hydroxyle, méthyle, méthoxy ;
R⁴ peut représenter hydrogène, méthyle, éthyle ;
R⁵ peut représenter hydrogène, méthyle, éthyle ;
R⁶ peut représenter hydrogène, méthyle, éthyle ;
R⁷ peut représenter t-butyle, cyclohexyle, phényle, la cas échéant, substitué par R⁹ et R¹⁰, qui peuvent être identiques ou différents, 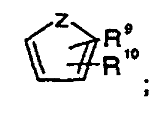
R⁸ peut représenter hydrogène, alkyle en C₁-C₄ ;
Z peut représenter oxygène, NH, soufre ;
R⁹ peut représenter hydrogène, méthyle, fluor, chlore, brome, méthoxy ;
R¹⁰ peut représenter hydrogène, méthyle, fluor, chlore, brome, méthoxy,
**caractérisé en ce que**
a) on soumet un cyanure de benzyle de la formule générale 32, dans laquelle R₃₀ représente un radical alkyle en C₁-C₄, avec un ester d'acide halogénocarboxylique de la formule générale 33, dans laquelle R₄₀ représente alkyle en C₁-C₈ ou benzyle, en présence d'un alkylhalogénosilane, de préférence un trialkylchlorosilane et de manière particulièrement préférée, le triméthylchlorosilane, et de la poudre de zinc dans un solvant inerte, de préférence dans un éther ou dans un halogénohydrocarbure et de manière particulièrement préférée, avec du dichlorométhane, et en présence d'un agent de réduction sélectif concernant la réduction des fonctions imino, de préférence en présence d'un dérivé d'un alkylborohydrure et de manière particulièrement préférée, en présence de cyanoborohydrure de sodium, aux conditions d'une réaction de Reformatsky et le dérivé ester d'acide carboxylique résultant de la formule générale 34 est isolé, et 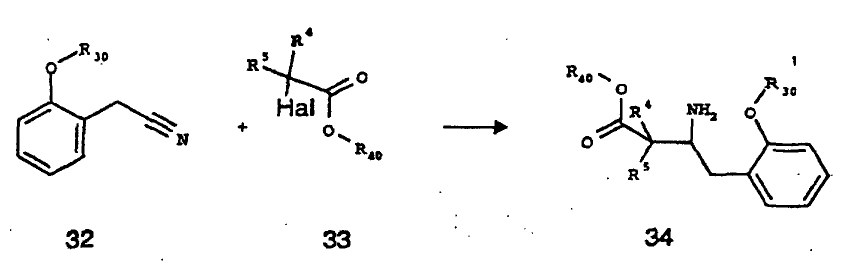
b) le dérivé ester d'acide carboxylique de la formule générale 34 est soumis aux conditions d'une réaction d'addition de Michael avec un ester d'acide acrylique, dans lequel, dans le composant alcool, R₅₀ signifie un radical alkyle en C₁-C₈ ou un radical benzyle, dans un solvant inerte dans les conditions de réaction choisies, de préférence dans un alcanol et de manière particulièrement préférée, dans l'éthanol, et le produit d'addition de Michael résultant de la formule générale 35 est isolé, 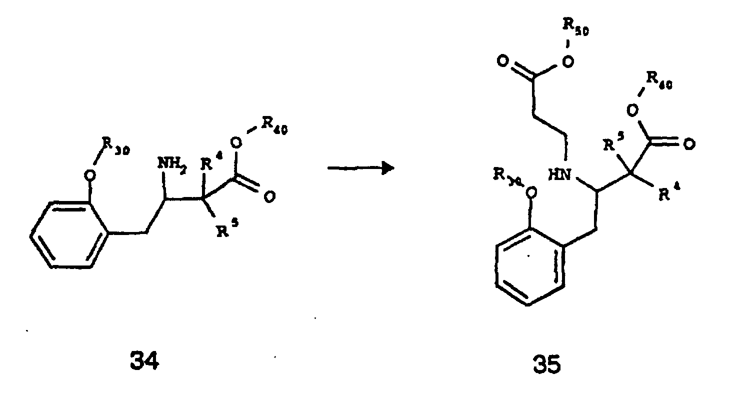 et
c) le dérivé diester d'acide carboxylique ainsi préparé de la formule générale 35 est soumis aux conditions d'une condensation d'ester de Dieckmann, dans un solvant inerte, de préférence dans un hydrocarbure aliphatique ou aromatique et de manière particulièrement préférée, dans le toluène, en présence d'un composé réactif basique, de préférence en présence d'un alcoolate alcalin d'un alcanol en C₁-C₄ ramifié ou non ramifié et de manière particulièrement préférée, en présence de t-butanolate de potassium, et le dérivé pipéridone résultant de la formule générale 36 est isolé : 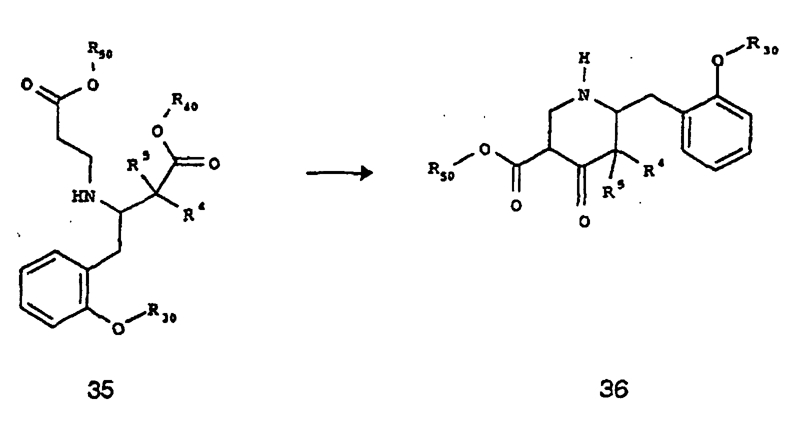 et,
d) le dérivé pipéridone 36 est saponifié et décarboxylé en conditions acides ou alcalines, de préférence en présence d'un hydroxyde alcalin ou d'un acide minéral et de manière particulièrement préférée, en présence d'hydroxyde de sodium, dans un solvant ou un mélange de solvants polaires, de préférence dans un mélange d'un alcanol C₁-C₄ linéaire ou ramifié et d'eau et de manière particulièrement préférée, dans un mélange éthanol-eau, avec chauffage en le dérivé ester de pipéridone correspondant de la formule générale 37, on isole et le cas échéant, on prépare le sel d'addition acide correspondant avec un acide et on isole : 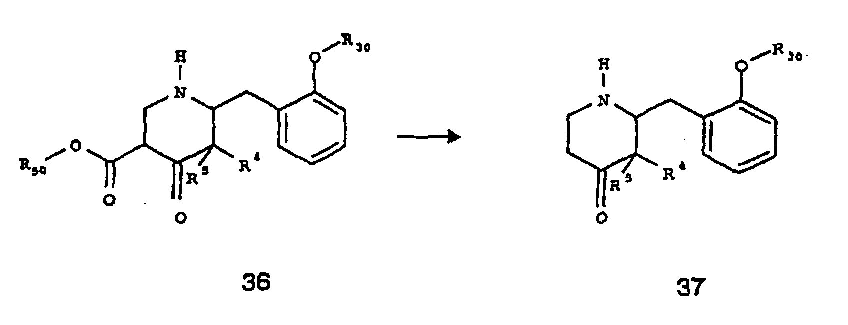 et,
e) si souhaité, concernant la séparation des énantiomères, le mélange de stéréoisomères ainsi obtenu, est dissous dans un milieu de réaction inerte, le cas échéant après libération de chaque base libre énantiomère, on additionne un stéréoisomère approprié d'un acide organique approprié pour former un sel avec un stéréoisomère du mélange d'énantiomères, le stéréoisomère souhaité est isolé sous forme de son sel d'addition avec l'acide optiquement actif, 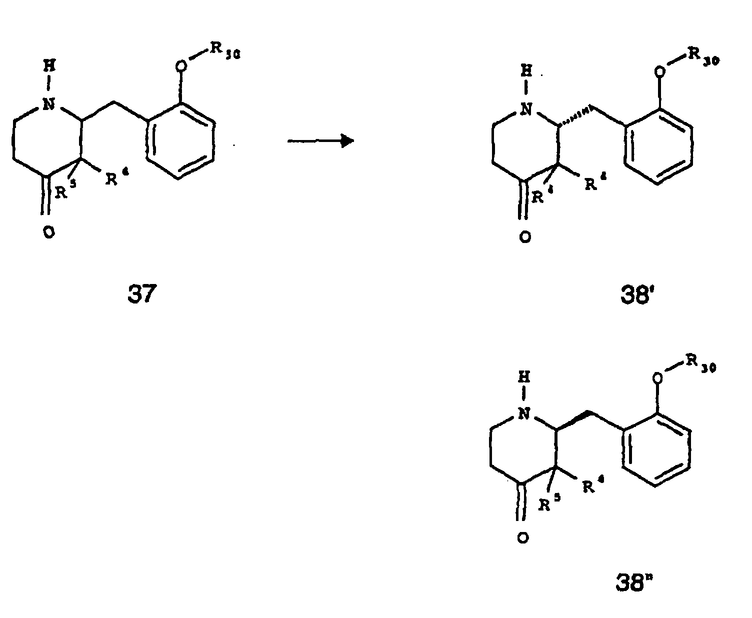 et
f) le stéréoisomère pur 38' ou 38'' ainsi obtenu ou le mélange d'isomère 38 est soumis à une réaction de Wittig, après libération du sel d'addition acide énantiomériquement pur, dans un solvant inerte, avec un réactif de Wittig générant un groupement CH₂= ou CH₃-CH=, de préférence avec un halogénure d'éthyltriphénylphosphonium ou avec un halogénure de méthyltriphénylphosphonium, de manière particulièrement préférée avec le bromure de méthyltriphénylphosphonium ou le bromure d'éthyltriphénylphosphonium, en présence d'un composé basique, de préférence en présence d'un alcoolate alcalin, de manière particulièrement préférée, le t-butanolate de potassium, dans un milieu de réaction inerte, de préférence dans un éther cyclique, de manière particulièrement préférée, dans le tétrahydrofuranne, et le produit de réaction du type 39 ou le stéréoisomère correspondant est isolé, le cas échéant sous forme d'un sel d'addition acide, 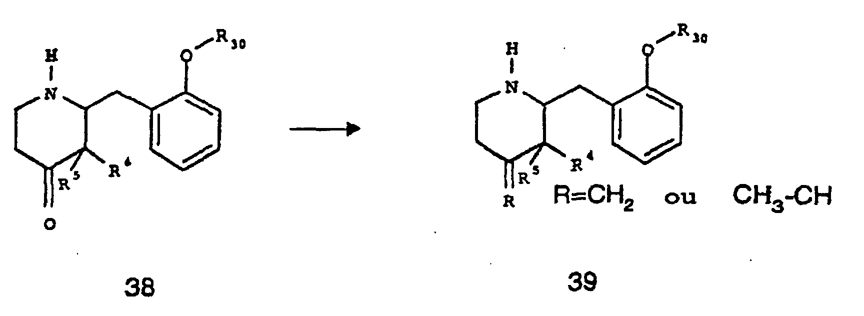 et,
g) l'alcène 39 résultant de la réaction de Wittig, est le cas échéant, d'abord libéré de son sel d'addition acide et la base libre du type 39 est dissoute dans un solvant organique, de préférence dans un hydrocarbure halogéné et de manière particulièrement préférée, dans le dichlorométhane et on soumet à une réaction de formylation sur l'azote de la pipéridine, avec un agent de formylation, de préférence le formiate de n-butyle et on isole le produit de réaction du type 40 ou le stéréoisomère correspondant : 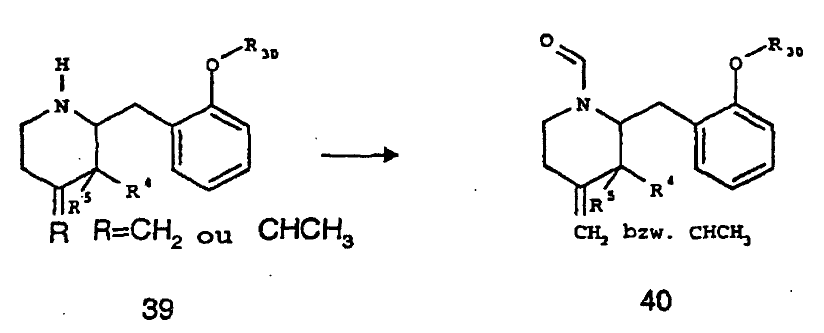 et,
h) le composé formyle 40 ainsi obtenu, ou le stéréoisomère correspondant est dissous, avec un acide minéral ou avec un acide de Lewis, de préférence le chlorure d'aluminium (III), dans un solvant inerte, de préférence dans un hydrocarbure halogéné et de manière particulièrement préférée, dans le dichlorométhane, on fait réagir et on isole le produit de cyclisation résultant de cette réaction du type 41, 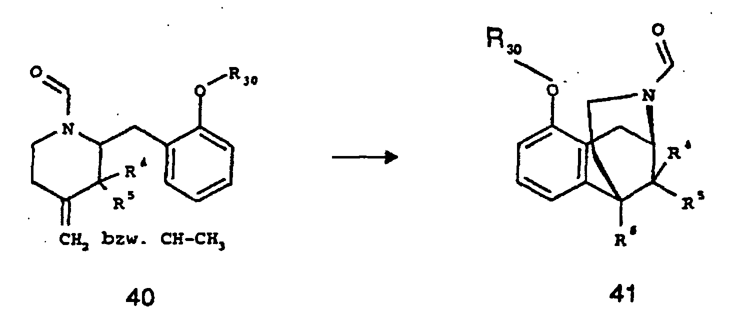 et
i) le dérivé benzomorphane résultant de la réaction de cyclisation est dissous dans un solvant polaire, de préférence dans un alcanol en C₁-C₄, de manière particulièrement préférée, dans le n-propanol, et on fait réagir avec un composé acide, de préférence avec la solution aqueuse d'un acide minéral et de manière particulièrement préférée, avec l'acide chlorhydrique concentré, et le norbenzomorphane déformylé résultant de cette réaction du type 42, est isolé le cas échéant sous forme de son sel d'addition acide, 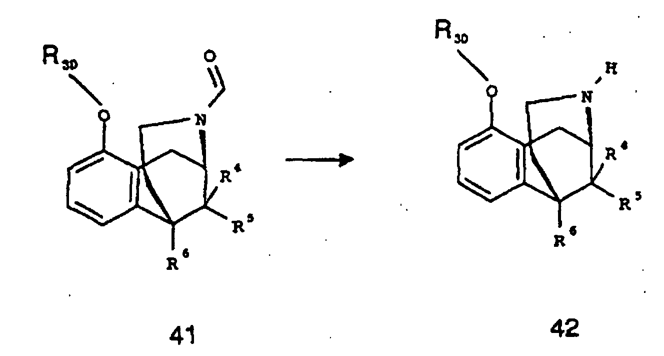 et
j) si aucune séparation des stéréoisomères n'a encore été réalisée à cette étape, on sépare les stéréoisomères de manière connue en soi et après libération de la base libre de benzomorphane, le phénoléther est clivé avec un composé acide, de préférence avec un acide minéral et de manière particulièrement préférée, avec l'acide bromhydrique et le produit de clivage de type 43 est isolé 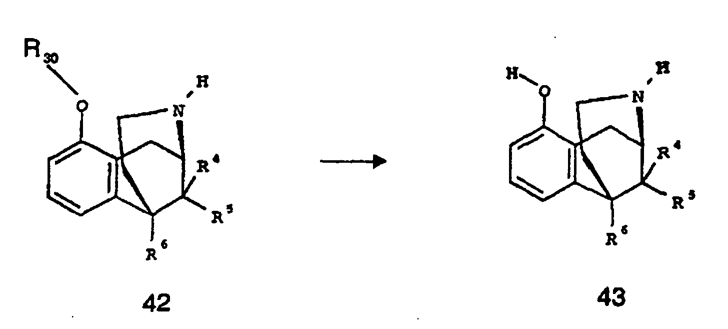
k) on fait réagir avec un composé du type Z-CHR₈-R', où Z signifie un groupe partant substituable par un azote d'amine secondaire, de préférence halogène, comme chlore, brome, iode ou un sulfonate organique, de préférence le trifluorométhanesulfonate et R' a la signification -CR¹R²XR⁷, 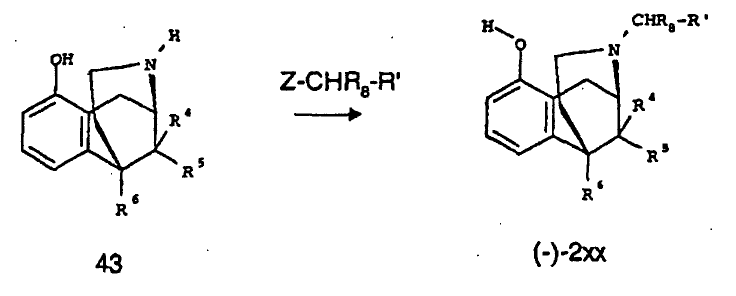 ou
on fait réagir avec un composé du type YC(O)R', où Y représente un groupe partant substituable par un azote d'amine secondaire, de préférence halogène, comme chlore, brome, iode ou un sulfonate organique, de préférence le trifluorométhanesulfonate et R' a la signification -CR¹R²XR⁷, et ensuite, le composé carbonylé est réduit en le composé (-)-2xx 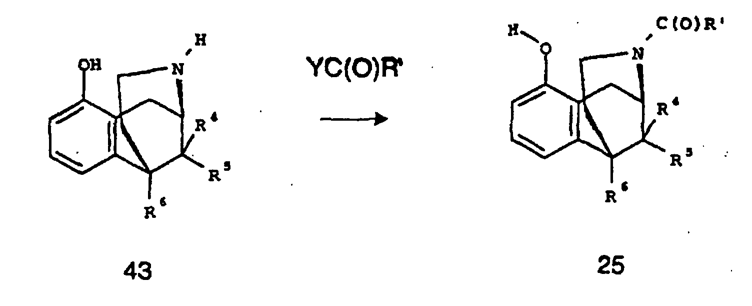 ou
on fait réagir avec un aldéhyde de la formule générale HC(O)-R', où R' a la signification -CR¹R²XR⁷, et la base de Schiff résultante 26 est réduite en le composé (-)-2xx 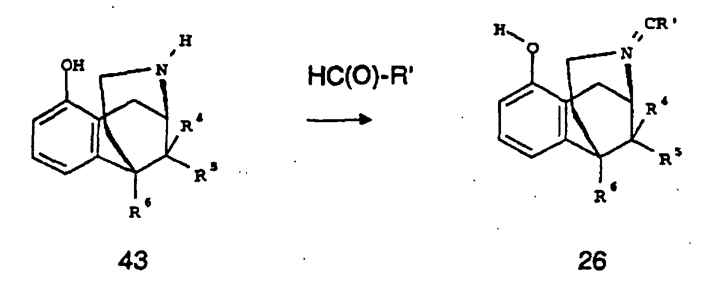 et
l) le cas échéant, dans le cadre d'une substitution électrophile, on introduit le substituant R³

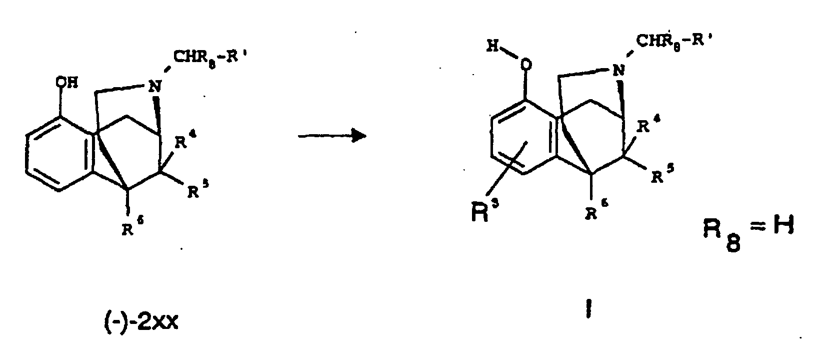

6. Composition pharmaceutique **caractérisée par** une teneur en un des composés selon l'une des revendications 1 à 4 et leurs sels d'addition acide, en plus d'auxiliaires et de supports usuels.

7. Composition pharmaceutique selon la revendication 6, **caractérisée en ce qu'**elle est formulée comme une solution pour injection.

8. Composition pharmaceutique selon la revendication 7, **caractérisée en ce que** la teneur en agent actif, sur base de la masse totale de la composition pharmaceutique se situe dans un intervalle allant de 0,001 à 20% en poids.

9. Composition pharmaceutique selon la revendication 8, **caractérisée en ce que** la teneur en agent actif, sur base de la masse totale de la composition pharmaceutique se situe dans un intervalle allant de 0,001 à 10% en poids.

10. Composition pharmaceutique selon la revendication 9, **caractérisé en ce que** la teneur en agent actif, sur base de la masse totale de la composition pharmaceutique se situe dans un intervalle allant de 0,01 à 5% en poids.

11. Utilisation des composés selon l'une des revendications 1 à 4, comme agent pharmaceutique.

12. Utilisation de composés selon l'une des revendications 1 à 4, pour la préparation d'un agent pharmaceutique pour le traitement thérapeutique d'ischémies cérébrales de différentes genèse, de maladies neurodégénératives, de l'épilepsie, de l'hypoglycémie, de l'hypoxie, de l'anoxie, d'un traumatisme cérébral, d'un oedème cérébral, d'un choc cérébral, d'une asphyxie périnatale, de la sclérose amylotrope latérale, de la maladie de Huntington, de la maladie d'Alzheimer, de la maladie de Parkinson, du désordre bipolaire, de la cyclothymie, de l'hypotonie, le l'infarctus cardiaque, des troubles du rythme cardiaque, de l'angine de poitrine, des douleurs pour l'anesthésie ou l'anesthésie locale.
